# EUROPÄISCHE PATENTANMELDUNG

(11) **EP 4 129 313 A2**
(43) Veröffentlichungstag der Anmeldung: **08.02.2023**
(21) Anmeldenummer: 22187665.9
(22) Anmeldetag: 28.07.2022
(51) Int. Cl.: A61K 36/185, A61K 35/618, A61P 17/00, A61K 9/08, A61K 9/20

(54) **PHARMAZEUTISCHE ZUSAMMENSETZUNG ZUR BEHANDLUNG VON NEURODERMITIS**

(30) Priorität: 03.08.2021 DE 102021120213
(71) Anmelder: Theis Cornelsen, Theis, 24229 Dänischenhagen (DE)
(72) Erfinder: Theis Cornelsen, Theis, 24229 Dänischenhagen (DE)
(74) Vertreter: RGTH

(57) **Zusammenfassung**

Die Erfindung betrifft eine pharmazeutische Zusammensetzung, umfassend Passionsblumenkraut in Form eines Trockenextrakts als einen Wirkstoff und/oder getrocknetes Miesmuschelfußkonzentrat als weiteren Wirkstoff zur Verwendung in der Behandlung von Neurodermitis durch orale oder topische Verabreichung, mit der Maßgabe, dass Passionsblumenkraut in Form eines Trockenextrakts nur zusammen mit getrocknetem Miesmuschelfußkonzentrat topisch verabreicht wird. Die Wirkung ist zum Teil bislang bekannten klassischen Arzneimitteln überlegen, da die aus dem Stand der Technik bekannten Nebenwirkungen nicht auftreten, und die natürliche Mikroflora der Haut und der Schleimhäute nicht geschädigt, sondern vielmehr intakt erhalten bleibt. Außerdem wird das Erscheinungsbild der Haut deutlich verbessert.

## Beschreibung

Die Erfindung betrifft eine pharmazeutische Zusammensetzung zur Behandlung von Neurodermitis.

### Hintergrund zur Erfindung

Eine Vielzahl von pflanzlichen Arzneimitteln, sogenannte "Phytopharmaka", ist bereits bekannt, deren Wirksamkeit in der Regel auf mehreren pflanzlichen Inhaltsstoffen beruht. Pflanzliche Arzneimittel werden auf Basis naturwissenschaftlicher Erkenntnisse zur Behandlung von Krankheiten eingesetzt (Phytotherapie) und müssen dieselben Anforderungen erfüllen wie die klassischen chemisch-synthetischen Arzneimittel.

Naturheilverfahren haben dabei neben oder in Ergänzung zur klassischen Medizin eine zunehmende Bedeutung erlangt. Heilkräuter und Heilpflanzen, auch als Arzneipflanzen bezeichnet, werden zu Heilzwecken oder zur Linderung und Behandlung von zahlreichen Krankheiten zur innerlichen oder äußerlichen Anwendung eingesetzt. Pflanzliche Arzneimittel können in unterschiedlichsten Formen zum Einsatz kommen, wie beispielweise Granulaten, Tabletten, Kapseln, Dragees, Lotionen, Teezubereitungen, Badezusätzen, in Kosmetika, Nahrungsergänzungsmitteln, ätherischen Ölen und v.a.m. Diese haben grundsätzlich dieselben Risiken wie alle Arzneimittel, sind im Vergleich mit den chemisch-synthetischen Wirkstoffen aber in der Regel besser verträglich.

Im Gegensatz zu chemisch-synthetischen Wirkstoffen in klassischen Arzneimitteln, wo nur ein oder wenige Wirkstoffe vorliegen, enthalten pflanzliche Arzneimittel in der Regel einen Pflanzenextrakt, der sich aus vielen verschiedenen Einzelsubstanzen zusammensetzt, die zusammen die Wirkung bestimmen.

Die im pflanzlichen Arzneimittel enthaltenen Wirkstoffe können auch die naturbelassene Pflanze oder deren Teile an sich darstellen, die sowohl unverarbeitet oder in geeigneter Weise verarbeitet, beispielweise zerkleinert, vorliegen kann. Das pflanzliche Arzneimittel basiert jedoch zumeist auf dem Extrakt der Pflanze, ist insbesondere ein wässriger Auszug (Mazerat), wässerig-alkoholischer Auszug oder alkoholischer Auszug der ganzen Pflanze oder Teilen hiervon. Hierfür können frische oder auch getrocknete Pflanzen oder Pflanzenteile zum Einsatz kommen. Die Pflanzenteile, die hierfür geeignet sind, hängen von der ausgewählten Arzneipflanze ab und sind zum Beispiel Blüten, Knollen, Früchte, Blätter, Stängel und/oder Wurzeln der Pflanze. Die gewonnenen Wirkstoffe stellen das Ausgangsmaterial für ein pflanzliches Arzneimittel dar und hieraus können verschiedene Darreichungsformen hergestellt werden.

Bekannt ist weiterhin, dass Heilpflanzen aufgrund ihrer verschiedenen Inhaltsstoffe eine Heilwirkung bei völlig verschiedenen Erkrankungen und Beschwerden zeigen können. Zur Erläuterung seien einige Beispiele angeführt:
So ist Aloe vera ein anerkanntes Mittel gegen Verstopfung, das als Gel oder Saft auch äußerlich zur Wundheilung dienen kann.

Die Wurzeln von Süßholz dienen als Heilmittel bei Magengeschwüren, Atemwegsentzündungen und anderen Beschwerden.

Ein sehr bekanntes pflanzliches Arzneimittel ist das Johanniskraut. Dieses wird aus den getrockneten Triebspitzen des echten Johanniskrauts (Hypericum perforatum) in Form eines Gesamtextrakts, bestehend aus mehreren Wirkstoffen, gewonnen. Es weist eine antibakterielle und antivirale Wirkung auf und ist auch für seine antidepressive Wirkung bekannt geworden, die in klinischen Tests bestätigt wurde. Der tatsächliche Wirkmechanismus ist jedoch nach wie vor unklar.

Es gibt einen wachsenden Bedarf an pflanzlichen Arzneimitteln, so dass weiterhin intensiv auf diesem Gebiet geforscht wird.

Zu den klassischen Heilpflanzen gehört auch die Passionsblume. Die Passionsblume Passiflora incarnata L. gehört zu den mehr als 500 Arten der Familie der Passionsblumengewächse und stellt eine ausdauernde und kletternde Staude dar, die unter anderem in den USA heimisch ist. Es ist eine bekannte Zierpflanze. Als pflanzliches Arzneimittel findet das Passionsblumenkraut (Passiflorae herba) bereits Verwendung. Es besteht aus den getrockneten, zerkleinerten oder geschnittenen, oberirdischen Teilen von Passiflora incarnata L. Blüten und Früchte können vorhanden sein. Das Kraut der Passionsblume bezeichnet daher sämtliche Pflanzenteile außer der Wurzel.

Das Passionsblumenkraut wird auch als Nahrungsergänzungsmittel eingesetzt.

Passionsblumenkraut und daraus hergestellte Zubereitungen haben beruhigende, angstlösende und krampflösende Eigenschaften. Die Präparate von Passionsblumenkraut werden unter anderem als Sedativum zur Beruhigung, zur Behandlung von Spannungszuständen, innerer Unruhe, Einschlafschwierigkeiten, Reizbarkeit und Nervosität eingesetzt. Eine ähnliche Wirkung wird den Heilpflanzen Baldrian, Melisse, Lavendel und Hopfen zugeschrieben, die auch in Kombinationspräparaten mit Passionsblumenkraut als Beruhigungsmittel zum Einsatz kommen.

Neben einer Wirkung gegen Unruhe, Angstzustände und Schlafstörungen soll auch eine Wirksamkeit bei Leberbeschwerden vorliegen. Außerdem ist eine Auftragung des Pulvers zur lokalen Wundheilung bekannt.

Inhaltsstoffe von getrocknetem Passionsblumenkraut sind unter anderem Flavonoide, wie Isovitexin, Isoorientin, Schaftosid, Cumarine, Glykoside, essentielle Fettsäuren, ätherisches Öl und Kohlenhydrate.

Passionsblumenkraut wird in unterschiedlichen Monopräparaten im Handel angeboten und ist beispielweise in Form von Dragees, Extrakten, Tropfen, Tee, Pulvern oder anderen Zubereitungsformen erhältlich. Bekannte Monopräparate auf Basis von Passionsblumenkraut-Trockenextrakt sind zum Beispiel "Valverde^{®} Beruhigung" und der Sidroga^{®}-Beruhigungstee. Getrocknetes Passionsblumenkraut ist als Offenware auch in Apotheken und Drogerien erhältlich.

Die Präparate werden in der Regel zwei- bis viermal täglich eingenommen. Zu den möglichen unerwünschten Wirkungen gehören selten auftretende allergische Reaktionen.

Die Verwendung von Passionsblumenkraut in Verbindung mit der Behandlung von Hauterkrankungen durch orale Verabreichung ist jedoch bislang nicht bekannt geworden.

Neurodermitis, auch bezeichnet als atopische Dermatitis oder atopisches Ekzem, ist die häufigste chronische Hauterkrankung, wobei Menschen in jedem Alter betroffen sind. Es ist eine chronisch entzündliche, mit Juckreiz einhergehende Hauterkrankung. Charakteristisch sind Juckreiz (Pruritus), eine gestörte Hautbarriere, die Veranlagung zu Allergien und chronischen Entzündungen der Haut sowie eine gestörte Hautflora (Mikrobiom).

Die Erkrankung beginnt häufig im Säuglings- und Kindesalter und verläuft typischerweise in Schüben, wobei beschwerdearme oder -freie Phasen sich mit Krankheitsphasen abwechseln. Die gestörte Hautbarriere und herabgesetzte Schutzfunktion der Haut bedingen eine stark erhöhte Empfindlichkeit gegenüber Irritationen und unspezifischen Reizen, so dass die Haut oft trocken, schuppig und gerötet ist, zudem juckt die Haut stark. Der Kontakt mit Keimen oder auch physikalischen oder chemischen Reizen kann leicht zu Entzündungen führen. Bakterien, wie Staphylokokken, Viren, wie Herpes, oder auch Pilze können derartige Infektionen noch verstärken.

Es ist daher eine chronisch entzündliche, aber nicht ansteckende Hauterkrankung, für die es häufig keine Heilung gibt, jedoch viele Therapiemöglichkeiten. Ziel der Therapie ist es, die Symptome zum Verschwinden zu bringen oder zumindest abzuschwächen und zu lindern und symptomfreie Phasen zu verlängern.

Die Ursachen von Neurodermitis gehen auf verschiedene Faktoren zurück. So kann die erbliche Vorbelastung eine Rolle spielen, da Gene identifiziert wurden, die bei der Entstehung von Neurodermitis unterstützend wirken, so dass die Haut ihre Barrierefunktion nicht richtig entwickeln kann. Wenn ungünstige Umwelteinflüsse hinzukommen, kann die Erkrankung dann ausbrechen.

Typische Symptome von Neurodermitis sind daher ganz allgemein trockene Haut mit geröteten entzündeten Stellen (Ekzeme), die meist sehr stark jucken; flächenhafte Verdickung und Vergröberung der Haut (Lichenifikation) und Knötchen und Pusteln. Der starke Juckreiz ist sehr störend und kann zu Schlafstörungen führen.

Neben der Kenntnis und Meidung individueller Provokationsfaktoren, wie Allergene, Stress, raue Bekleidung (z.B. Wolle), ausgedehnte oder heiße Bäder, intensive Sonnenbestrahlung, Schweiß, Tabakrauch, Autoabgase oder dergleichen, und einer besonders sorgfältigen Pflege der Haut, ist häufig auch eine medikamentöse Behandlung erforderlich. Hierzu werden bislang nur klassische synthetisch-chemische Arzneimittel eingesetzt.

Antibiotika stellen keine geeignete Behandlung dar. Mittel der Wahl sind entzündungshemmende Arzneimittel, insbesondere Glukokortikosteroide ("Kortisonpräparate"). Sie werden üblicherweise als Cremes, Salben oder auch als Tabletten, zumeist aber nur kurzfristig, verabreicht. Die Wirkung kann erst verzögert nach mehreren Tagen einsetzen. Glukokortikosteroide führen bei langer Anwendung dazu, dass die Haut dünner wird oder es resultieren unerwünschte Hautveränderungen. Diese dürfen daher nicht regelmäßig täglich angewandt werden, zudem sollten Bereiche mit dünner Haut, wenn überhaupt, dann nur kurzfristig behandelt werden.

Eine weitere Behandlungsmöglichkeit sind Calcineurin-Inhibitoren. Diese sollen auch bei ständiger Anwendung die Haut nicht ausdünnen, können jedoch kurzfristig zu einer Verstärkung des Juckreizes führen. Das Medikament ist für Säuglinge nicht geeignet.

Bekannt geworden ist auch die Behandlung von mittelschweren bis schweren Fällen von Neurodermitis mit dem IL-4-Rezeptor-Blocker Dupilumab, das alle zwei Wochen gespritzt wird. Als Nebenwirkungen können jedoch Augenprobleme mit milden Reizungen bis zu stärkeren Entzündungen auftreten.

Als weitere Behandlungsmöglichkeit ist die Verabreichung von Ciclosporin A bekannt, dass zwar einen raschen Wirkungseintritt zeigen kann, aber nach dem Absetzen regelmäßig zu einem Rebound-Effekt führt, d.h. einem verstärkten Wiederauftreten der Symptome der medikamentös behandelten Erkrankung nach Absetzen des Arzneimittels. Nebenwirkungen sind Blutdruckerhöhung, Nephrotoxizität und ein stark erhöhtes Risiko für weißen Hautkrebs.

Weiterhin sind aus dem Stand der Technik folgende Vorschläge zur Behandlung von Hauterkrankungen bekannt geworden:
Die US 2016/0 074 312 A1 bezieht sich auf eine Zusammensetzung, die ein Peptid und einen Zuckerextrakt aus Passionsblumensamen als Wirkstoff und gegebenenfalls einen geeigneten Hilfsstoff enthält, wobei der Peptid- und Zuckerextrakt durch enzymatische Hydrolyse in Gegenwart von mindestens einer Protease und mindestens einer Carbohydrase erhalten wird. Die Zusammensetzung wird kosmetisch, pharmazeutisch, dermatologisch oder als Nahrungsergänzungsmittel verwendet. Es wird unter einer Vielzahl von Einsatzmöglichkeiten auch die Behandlung von atopischer Dermatitis erwähnt. Die Inhaltsstoffe der Passionsblumen-Samen werden chemisch, biochemisch bzw. biotechnologisch verändert: In Beispiel 1 der US 2016/0 074 312 A1 wird eine enzymatische Hydrolyse durchgeführt, dann eine Hydrolyse mit einer Säureprotease, gefolgt von einer Wärmebehandlung, bei der die Enzyme denaturiert werden. Diese Verfahren bedeuten erhebliche Veränderungen, chemische Umwandlungen und z.T. sogar eine Zerstörung der natürlichen Inhaltsstoffe. Dies steht im Gegensatz zur vorliegenden Erfindung, wonach die natürlichen Inhaltsstoffe erhalten bleiben sollen, keinerlei chemische, biochemische oder biotechnologische Behandlungen der Pflanzenteile erfolgen und zudem der Passionsblumen-Extrakt auf möglichst schonende Weise gewonnen wird, um die Inhaltsstoffe möglichst nicht zu verändern. Zugesetzte Proteasen und Carbohydrasen sollen im Passionsblumen-Trockenextrakt der vorliegenden Erfindung zudem nicht vorliegen.

Die US 2019/0 209 631 A1 beschreibt einen Lipid-Extrakt von Passiflora-Samen, Passiflora incarnata, Passiflora edulis oder Mischungen hiervon. Es ist ein konzentriertes Öl, wobei unter einer Vielzahl von möglichen Verwendungen auch die atopische Dermatitis erwähnt wird. Der Lipid-Extrakt wird durch Molekulardestillation von rohem oder raffiniertem Passiflora-Öl erhalten. Ein Lipid-Extrakt weist nur unpolare, in Öl lösliche Inhaltsstoffe auf und hat daher eine völlig andere Zusammensetzung an Inhaltsstoffen als ein wässriger/alkoholhaltiger Extrakt, der nur polare und Wasser-/Alkohol-lösliche Inhaltsstoffe aufweist; es liegt damit generell ein völlig anderes Wirkungsspektrum vor. Ein Trockenextrakt des Passionsblumenkrauts, der aus einem Lipid-Extrakt erhältlich ist, wird in der vorliegenden Erfindung nicht eingesetzt.

Ferner beschreibt Manez, S. et al., Selected Extracts from Medicinal Plants as Anti-Inflammatory Agents, in: Planta Med., 1990, Bd. 56, S. 656, einen wässrigen Extrakt von Perna canaliculus (Grünschalmuscheln, Grünlipp-Muscheln oder Neuseeland-Miesmuscheln), wobei ein wässriger Extrakt von der ganzen Muschel gewonnen wird. Es werden in Pfoten von Schweizer Albinomäusen durch subkutane Injektion von Carrageenan-Lösung Ödeme erzeugt. Um die Ödeme zu verhindern, wurde den Mäusen der Extrakt intraperitoneal 1 h vor der künstlichen Ödem-Erzeugug gespritzt. Eine orale oder topische Verabreichung ist nicht beschrieben. Es wird ein wässriger Extrakt der Neuseeland-Miesmuscheln verwendet und kein Konzentrat. Im verwendeten wässrigen Extrakt sind nur wasserlösliche Inhaltsstoffe enthalten. Die Behandlung von auftretenden Ödemen, d.h. Flüssigkeitsansammlungen im Gewebe, steht in keinem Zusammenhang mit einer Neurodermitis-Behandlung der Haut. Zudem werden Neuseeland-Miesmuscheln erfindungsgemäß bevorzugt nicht eingesetzt.

Weiterhin offenbart die US 2020/0 353 024 A1 eine entzündungshemmende Zusammensetzung von Candida albicans, die hitzegetöteten Candida albicans in Kombination mit einer neuroprotektiven Ergänzung aufweist. Die neuroprotektive Ergänzung ist beispielweise ein positiver allosterischer Modulator des GABAA-Rezeptors, wie ein Phytomedikament, wobei unter einer Vielzahl von möglichen Pflanzen auch Passionsblumen als Ganzes oder Pflanzenextrakt angeführt wird. Die Zusammensetzung wird u.a. auch bei atopischer Dermatitis verabreicht. Candida albicans, insbesondere hitzegetöteten Candida albicans, ist in der pharamzeutischen Zusammensetzung der vorliegenden Erfindung nicht enthalten.

Ferner beschreibt die WO 00/53 198 A1 die Verwendung eines Lipid-Extrakts von Perna canaliculus oder Mytulis edulis, der in einer großen Reihe an Krankheiten und Störungen beispielweise bei Dermatitis oder atopischem Ekzem oral oder auch topisch verabreicht wird. Der Lipid-Extrakt wird durch superkritische Fluid-Extraktion aus gefriergetrocknetem Muschelpulver mit CO₂ erhalten. Es handelt sich nur um die Öllöslichen Inhaltsstoffe, wobei fraglich ist, ob diese Inhaltsstoffe die Prozedur der Gefriertrocknung unbeschadet überstehen. In den Beispielen wird zudem nur die Wirksamkeit bei Bronchialasthma demonstriert. Ein Lipid-Extrakt der Miesmuschel bzw. des Miesmuschelfußes wird in der vorliegenden Erfindung nicht verwendet.

Die WO 00/71 140 A2 bezieht sich auf eine Zusammensetzung, die N,N-Dimethylglycin und Perna canaliculus beispielweise als gefriergetrocknete zerkleinerte ganze Muschel enthält. Die Zusammensetzung wird u.v.a. auch gegen Dermatitis oral verabreicht. Weder der Miesmuschelfuß zur Herstellung des Konzentrats, noch das Miesmuschelfuß-Konzentrat in der pharmazeutischen Zusammensetzung der vorliegenden Erfindung sollen gefriergetrocknet werden. Ferner wird N,N-Dimethylglycin in der pharmazeutischen Zusammensetzung der vorliegenden Erfindung nicht eingesetzt.

Schließlich offenbart die CN 104 336 727 A eine Art von Getränk mit Milcharoma, das z. B. zur Verbesserung der Sehkraft verwendet wird und der traditionellen chinesischen Medizin entstammt. Es werden neben dem Saft der Passionsblume auch getrocknete Muscheln eingesetzt und z.T. auch eine Reihe an sehr exotischen Zutaten, wie Dendrobium candidum (eine Orchideenart), Mandarinenschalen-Saft, Schweineleber, Granatapfelblatt, Lotus, Erdnuss-Reis, Lilie und dergleichen. In der vorliegenden Erfindung sind die einzigen Wirksubstanzen Passionsblumen-Trockenextrakt und/oder Miesmuschelfuß-Konzentrat, weitere Wirksubstanzen sind in der pharmazeutischen Zusammensetzung nicht enthalten, sondern nur optional ein oder mehrere Hilfsstoffe.

Viele an Neurodermitis Erkrankte, insbesondere Kinder, empfinden es als mühsam, zeitraubend und auch unangenehm, wirkstoffhaltige Salben und Cremes manchmal über viele Jahre hinweg immer wieder auf die Haut auftragen zu müssen. Daher besteht ein großes Interesse und ein großer Bedarf für eine Behandlung von Neurodermitis in systemischer Art und Weise, insbesondere durch orale Verabreichung eines Arzneimittels. Weiterhin sollen auch bei lang andauernder Anwendung des Arzneimittels keine der aus dem Stand der Technik bekannten Nebenwirkungen auftreten. Insbesondere soll das Arzneimittel möglichst gut verträglich sein und nur sehr wenige Nebenwirkungen zeigen.

### Detaillierte Beschreibung der Erfindung

Die geschilderte Aufgabe wird durch eine pharmazeutische Zusammensetzung gelöst, umfassend
Passionsblumenkraut in Form eines Trockenextrakts als einen Wirkstoff und/oder getrocknetes Miesmuschelfuß-Konzentrat als weiteren Wirkstoff, zur Verwendung in der Behandlung von Neurodermitis durch orale oder topische Verabreichung,
wobei das Passionsblumenkraut in Form eines Trockenextrakts erhältlich ist durch
   - Ernten, Zerkleinern und Trocknen des Passionsblumenkrauts in Form der Pflanzenteile der Passionsblume, ohne Wurzel;
   - Durchführen einer Fest-Flüssig-Extraktion der getrockneten Pflanzenteile unter Verwendung von einem Extraktionsmittel, ausgewählt aus Wasser, Alkohol oder Alkohol-Wasser-Gemischen bei Raumtemperatur (25°C) oder einer Temperatur im Bereich zwischen Raumtemperatur (25°C) und 78°C, mit oder ohne Rühren und/oder Schütteln;
   - Abtrennen der Pflanzenteile vom Extraktionsmittel durch Dekantieren oder Filtrieren, und
   - Abdampfen des Extraktionsmittels unter Erhalt eines Trockenextrakts des Passionsblumenkrauts; und
wobei das getrocknete Miesmuschelfuß-Konzentrat zerkleinerten und getrockneten Miesmuschelfuß von Miesmuscheln darstellt, mit der Maßgabe, dass Passionsblumenkraut in Form eines Trockenextrakts nur zusammen mit getrocknetem Miesmuschelfuß-Konzentrat topisch verabreicht wird und dass Candida albicans und N,N-Dimethylglycin in der pharmazeutischen Zusammensetzung nicht enthalten sind.

Gemäß der vorliegenden Erfindung wird daher eine pharmazeutische Zusammensetzung bereitgestellt, die auf in der Natur vorkommenden Substanzen basiert, insbesondere auf Basis einer Heilpflanze und/oder tierischen Proteinen hergestellt werden kann. Die pharmazeutische Zusammensetzung kann folgende Wirkstoffe aufweisen:
- Passionsblumenkraut in Form eines Trockenextrakts;
- Passionsblumenkraut in Form eines Trockenextrakts und getrocknetes Miesmuschelfuß-Konzentrat; oder
- getrocknetes Miesmuschelfuß-Konzentrat.

Die pharmazeutische Zusammensetzung wird entweder oral oder topisch (durch Auftragen auf die Haut) verabreicht, wobei jedoch Passionsblumenkraut in Form eines Trockenextrakts nicht als alleiniger Wirkstoff in der pharmazeutischen Zusammensetzung topisch verabreicht wird; bei einer topischen Verabreichung wird das Passionsblumenkraut in Form eines Trockenextrakts stets zusammen mit getrocknetem Miesmuschelfuß-Konzentrat verabreicht. Jedoch kann getrocknetes Miesmuschelfuß-Konzentrat auch als einziger Wirkstoff in der pharmazeutischen Zusammensetzung topisch verabreicht werden. Zur oralen Verabreichung kommt sowohl Passionsblumenkraut in Form eines Trockenextrakts; eine Kombination von Passionsblumenkraut in Form eines Trockenextrakts und getrocknetes Miesmuschelfuß-Konzentrat; als auch getrocknetes Miesmuschelfuß-Konzentrat in Frage.

Ein Wirkstoff der erfindungsgemäßen pharmazeutischen Zusammensetzung ist daher ein Trockenextrakt des Passionsblumenkrauts. Der Trockenextrakt des Passionsblumenkrauts wird hier als "ein Wirkstoff" bezeichnet, obwohl eine Vielzahl von verschiedenen Substanzen enthalten ist. Da die Gesamtheit der extrahierten pflanzlichen Inhaltsstoffe die erfindungsgemäße Wirkung bereitstellt, wird dies vereinfacht als "ein Wirkstoff" beschrieben.

Das Passionsblumenkraut stellt die Pflanzenteile der Passionsblume (Passiflora incarnata L.), außer der Wurzel, dar. Es besteht daher die Möglichkeit von der Pflanze selbst sämtliche Pflanzenteile - außer der Wurzel - zu ernten, diese zu zerkleinern, beispielsweise in kleine Stücke zu schneiden, und zu trocknen. Bevorzugt sind die Passionsblumen aus kontrolliert biologischem Anbau. Alternativ ist getrocknetes Passionsblumenkraut auch im Handel erhältlich und kann beispielweise in einer Apotheke oder Drogerie erworben werden.

Aus dem getrockneten Passionsblumenkraut wird unter Verwendung eines Extraktionsmittels ein Extrakt hergestellt. Das Extraktionsmittel kann ausgewählt werden aus Wasser, Alkohol-Wasser-Gemischen oder Alkohol. Besonders bevorzugt sind Alkohol-Wasser-Gemische, insbesondere Ethanol-Wasser-Gemische mit einem Gehalt von 50% Ethanol (Vol./Vol.). Es kann bei Raumtemperatur (25°C) oder erhöhter Temperatur bis maximal 78°C (Siedepunkt von Ethanol) extrahiert werden. Zur Absenkung der Temperatur und schonenden Extraktion kann hierbei auch im Vakuum gearbeitet werden. Es wird daher eine Fest-Flüssig-Extraktion mit den festen Pflanzenteilen und dem flüssigen Extraktionsmittel durchgeführt. Die Pflanzenteile - frisch oder in getrockneter Form - können auch einfach nur im Extraktionsmittel über einen längeren Zeitraum mit oder ohne Rühren und/oder Schütteln stehen gelassen werden. Je nach den eingesetzten Mengen und der Art an getrocknetem Passionsblumenkraut, dem ausgewählten Extraktionsmittel und den Extraktionsbedingungen kann die Extraktion mehrere Stunden, Tage oder sogar mehrere Wochen dauern.

Der erhaltene flüssige Extrakt wird vom extrahierten Passionsblumenkraut getrennt, beispielsweise durch Dekantieren oder Filtrieren, und das enthaltene Lösungsmittel wird durch Ver- oder Abdampfen, beispielweise im Vakuum, entfernt. Hierdurch wird der Trockenextrakt des Passionsblumenkrauts erhalten, der als eine Grundlage für die pharmazeutische Zusammensetzung dient.

Der Trockenextrakt des Passionsblumenkrauts stellt ein Konzentrat der Pflanzeninhaltsstoffe in fester Form dar, das hygroskopisch ist und in Lösung lichtempfindlich sein kann. In der Regel werden Extrakte von 5:1 bis 7:1 oder auch bis zu 10:1 bereitgestellt. Dies bedeutet, dass bei einem 5:1 Extrakt: 5 Teil der Pflanze als 1 Teil des Trockenextrakts aus der Extraktion und dem Trocknen erhalten werden. Zum Beispiel werden bei einem 5:1 Extrakt: aus 5500 mg Passionsblumenkraut 1100 mg Extrakt erhalten.

Neben der Herstellung aus der frischen Pflanze kann ein entsprechendes Trockenextrakt auch direkt im Handel, beispielweise bei Apotheken oder Drogeriemärkten, erworben werden. Es hat sich herausgestellt, dass die im Handel erhältlichen Trockenextrakte hinsichtlich ihrer Qualität für die Herstellung einer pharmazeutischen Zusammensetzung zur Behandlung von Neurodermitis gut geeignet sind.

Von Bedeutung für die vorliegende Erfindung ist, dass die festen Pflanzenteile des Passionsblumenkrauts keiner die Inhaltsstoffe verändernden Behandlung, insbesondere keiner chemischen, biochemischen oder biotechnologischen Behandlung, wie beispielweise einer enzymatischen Hydrolyse, unterzogen werden, d.h. unbehandelt eingesetzt werden. Die Pflanzenteile aus der Natur werden als solche verwendet. Selbstverständlich kann man die Pflanzenteile kurz (wenige Minuten) mit Wasser waschen, um Schmutz zu entfernen; dies stellt jedoch keine Behandlung im Sinne der vorliegenden Erfindung dar, da die Pflanzeninhaltsstoffe hierdurch nicht verändert werden. Erfindungsgemäß sollen die natürlichen Inhaltsstoffe daher im Trockenextrakt nicht durch chemische oder biologische Zusätze verändert werden und erhalten bleiben. Aus den unbehandelten Pflanzenteilen werden dann der flüssige Extrakt und daraus dessen Trockenextrakt gewonnen, wobei der Passionsblumen-Extrakt auf möglichst schonende Weise gewonnen wird, um die Inhaltsstoffe nicht zu verändern. Eine chemische, biochemische oder biotechnologische Behandlung während der Herstellung des Trockenextrakts ist daher gänzlich unerwünscht und erfolgt nicht. Dies würde die Inhaltsstoffe der Pflanzenteile verändern und damit deren Wirksamkeit deutlich herabsetzen oder gar zerstören. Eine zusätzliche Wärme- oder Kälte-Behandlung während der Herstellung des Trockenextrakts außer der Fest-Flüssig-Extraktion des Passionsblumenkrauts, wie ein zusätzliches Erhitzen oder ein Gefriertrocken, soll ebenfalls nicht durchgeführt werden, um die Inhaltsstoffe nicht zu beeinträchtigen. Jede die Inhaltsstoffe beeinträchtigende Behandlung soll daher vermieden werden.

Durch mehrjährige Versuche konnte festgestellt werden, dass der Trockenextrakt des Passionsblumenkrauts als alleiniger Wirkstoff in einer pharmazeutischen Zusammensetzung zur Behandlung von Neurodermitis erfolgreich einsetzbar ist. Er kann oral verabreicht werden, eine topische Verabreichung als alleiniger Wirkstoff ist erfindungsgemäß ausgeschlossen.

Bei Versuchen mit einer pharmazeutischen Zusammensetzung, umfassend den Trockenextrakt des Passionsblumenkrauts als alleinigen Wirkstoff, zur oralen Verabreichung wurden bei der Behandlung von Neurodermitis eine verminderte Rötung und ein deutlich reduzierter Juckreiz festgestellt. Im Allgemeinen wurde eine deutliche Beruhigung der entzündeten Hautpartien beobachtet. Insgesamt wurde durch den Trockenextrakt des Passionsblumenkrauts eine deutliche Verbesserung des Hautbildes bei Neurodermitis erhalten. Damit wirkt der Trockenextrakt des Passionsblumenkrauts sich besonders positiv auf die Haut allgemein und insbesondere die von Neurodermitis befallene Haut aus - was völlig im Gegensatz zu den ansonsten verabreichten entzündungshemmenden Arzneimitteln, wie Glukokortikoiden, steht, die die Haut, insbesondere bei längerem Gebrauch, schädigen können.

Unerwünschte Nebenwirkungen wurden bei den mehrjährigen Versuchen nicht festgestellt.

Ein weiterer Wirkstoff der pharmazeutischen Zusammensetzung der vorliegenden Erfindung, der allein oder zusammen mit dem Trockenextrakt des Passionsblumenkrauts zum Einsatz kommen kann, ist ein getrocknetes Miesmuschelfuß-Konzentrat. Wie beim Trockenextrakt des Passionsblumenkrauts wird auch das getrocknete Miesmuschelfuß-Konzentrat hier einfach als "ein Wirkstoff" bezeichnet, obwohl mehrere verschiedene Substanzen, insbesondere Proteine, enthalten sind, diese jedoch in ihrer Gesamtheit die Wirkung bereitstellen, d.h. wie ein Wirkstoff wirken.

Das getrocknete Miesmuschelfuß-Konzentrat wird aus Miesmuscheln gewonnen. Miesmuscheln, die auch als Pfahlmuscheln oder Blaumuscheln bezeichnet werden, sind essbare Muscheln (Mytilidae), die in vielen Ländern als Delikatesse gelten. Sie sind für ihre blau-schwarze Schale und ihr festes Fleisch bekannt geworden. Der Name dieser Muschel stammt vom altdeutschen Wort für Moos, da die Miesmuscheln in ihrem Lebensraum auf dem Meeresboden große Teppiche ausbilden, welche optisch an Moos erinnern.

Der Lebensraum der Miesmuschel ist unter anderem in der Nordsee vor Dänemark, Belgien, den Niederlanden und den Atlantikküsten vor Frankreich, Spanien und Portugal. Ebenso findet man diese vor deutschen Küsten; im Wattenmeer der Nordseeküste bildet sie große Naturbänke aus. 95 Prozent der weltweiten Erzeugung von Miesmuscheln stammen aus Aquakulturen, wobei die größten Erzeuger China und die EU sind. Miesmuscheln aus Neuseeland, insbesondere Grünschalmuscheln, werden erfindungsgemäß bevorzugt nicht verwendet.

Man findet die Miesmuschel hauptsächlich in den Gezeiten- und Schelfbereichen der Meere. Die Muscheln besitzen ein Gehäuse, das aus zwei kalkhaltigen Klappen besteht, die zusammengehalten werden. Im Inneren der Muschel befindet sich der Körper des Weichtieres. Muscheln besitzen Kiemen und können daher dauerhaft unter Wasser leben. Damit erhalten sie den Sauerstoff und auch die Nährstoffe aus dem Wasser.

Im Lebensbereich der Miesmuscheln herrscht in der Regel eine starke Strömung. Um in diesen Regionen überleben zu können und nicht ins Meer hinausgespült zu werden, scheidet die Miesmuschel Fäden aus, mit denen sich diese festhält. Zwischen den Kiemen der Miesmuschel befindet sich ein muskulöser Fuß mit der Byssusdrüse. Diese Drüse stellt mit Hilfe von in der Miesmuschel enthaltenem Eiweiß die sogenannten Byssusfäden her; dies sind Fäden oder Filamente mit denen sich die Muschel festhalten kann. Die Miesmuscheln verwenden diese Fäden oder Filamente, um an Felsen; Booten oder anderen Oberflächen in den Gezeitenzonen auch bei starker Meeresströmungen zu haften.

Der Muschelfuß stellt daher ein besonderes Organ dar, mit dessen Hilfe sich die Muscheln festhalten und auch fortbewegen können.

In der vorliegenden Erfindung wird ein getrocknetes Miesmuschelfuß-Konzentrat als Wirkstoff eingesetzt. Insbesondere werden Miesmuscheln der Gattung Mytilus edulis verwendet. Das Miesmuschelfuß-Konzentrat wird direkt aus möglichst frischen Miesmuscheln durch Herauslösen des Fußes, Zerkleinern und Trocknen des gewonnenen Materials erhalten.

Bevorzugt stammen die Miesmuscheln, aus denen der Fuß entnommen wird, aus einem Naturschutzgebiet, wie beispielsweise das Naturschutzgebiet Wattenmeer vor Sylt, da dort besonders gute Umweltbedingungen vorliegen. Das erhaltene getrocknete Miesmuschelfuß-Konzentrat besteht hauptsächlich aus Proteinen, die sich durch besondere Eigenschaften auszeichnen, die bei der Behandlung von Neurodermitis zum Tragen kommen.

Von Bedeutung für die vorliegende Erfindung ist, dass der Miesmuschelfuß keiner die Inhaltsstoffe verändernden Behandlung, insbesondere keiner chemischen, biochemischen oder biotechnologischen Behandlung, wie beispielweise einer enzymatischen Hydrolyse, unterzogen wird, d.h. unbehandelt eingesetzt wird. Der Miesmuschelfuß aus der Natur wird als solcher in unbehandelter Form verwendet. Selbstverständlich kann man die Muschel mit Wasser waschen, um Schmutz zu entfernen; dies stellt jedoch keine unerwünschte Behandlung im Sinne der vorliegenden Erfindung dar, da die Inhaltsstoffe hierdurch nicht verändert werden. Erfindungsgemäß sollen die natürlichen Inhaltsstoffe daher im Miesmuschelfuß-Konzentrat auch nicht durch chemische oder biologische Zusätze verändert werden und erhalten bleiben. Das Konzentrat ist kein Extrakt, sondern enthält alle Inhaltsstoffe des Miesmuschelfußes in unbehandelter Form. Eine chemische, biochemische oder biotechnologische Behandlung vor oder während der Herstellung des Konzentrats ist daher gänzlich unerwünscht und erfolgt nicht. Dies würde die Inhaltsstoffe verändern und damit deren Wirksamkeit deutlich herabsetzen oder gar zerstören. Eine zusätzliche Wärme- oder Kälte-Behandlung vor oder während der Herstellung des Konzentrats, wie ein Erhitzen oder ein Gefriertrocken, soll ebenfalls nicht durchgeführt werden, um die Inhaltsstoffe nicht zu beeinträchtigen. Jede die Inhaltsstoffe beeinträchtigende Behandlung soll daher vermieden werden.

Durch mehrjährige Versuche konnte festgestellt werden, dass Miesmuschelfuß-Konzentrat als alleiniger Wirkstoff in einer pharmazeutischen Zusammensetzung zur Behandlung von Neurodermitis erfolgreich einsetzbar ist. Es kann oral oder topisch verabreicht werden. Die Versuche bestätigen, dass ein unter Neurodermitis leidender Patient, der mit einer pharmazeutischen Zusammensetzung, umfassend Miesmuschelfuß-Konzentrat, behandelt wird, eine deutliche Beruhigung der entzündeten Haut erfährt, zudem wird eine Stärkung der Festigkeit, Verminderung von Hautschuppungen sowie eine Glättung der Haut beobachtet. Insgesamt wird durch eine pharmazeutische Zusammensetzung, umfassend das Miesmuschelfuß-Konzentrat, eine deutliche Verbesserung des Hautbildes bei Neurodermitis erhalten. Eine pharmazeutische Zusammensetzung, umfassend das Miesmuschelfuß-Konzentrat als einzigen Wirkstoff, wirkt sich besonders positiv auf die Haut allgemein und insbesondere die von Neurodermitis befallenen Hautpartien aus - was völlig im Gegensatz zu den üblichen entzündungshemmenden Arzneimitteln, wie Glukokortikoiden steht, die die Haut, insbesondere bei längerem Gebrauch, schädigen können.

Unerwünschte Nebenwirkungen wurden bei den mehrjährigen Versuchen nicht festgestellt.

In der vorliegenden Erfindung kann auch ein Kombinationspräparat aus Trockenextrakt des Passionsblumenkrauts und getrocknetem Miesmuschelfuß-Konzentrat, oral oder topisch, zusammen in einer Dosierungsform (sogenannte Festdosiskombination, wobei die beiden Wirkstoffe, die kombiniert werden sollen, gleichzeitig in derselben pharmazeutischen Formulierung vorliegen) oder in zwei gleichen oder verschiedenen Dosierungsformen (freie Dosiskombinationen, wobei die beiden Wirkstoffe, die kombiniert werden sollen, in getrennten pharmazeutischen Formulierungen vorliegen) verabreicht werden.

Für die orale Verabreichung können beispielweise beide Wirkstoffe gleichzeitig in einer Kapsel oder Tablette vorliegen oder ein Wirkstoff liegt in einer Kapsel und der andere Wirkstoff liegt in einer Kapsel oder einer Tablette vor.

Für die topische Verabreichung können beispielweise beide Wirkstoffe gleichzeitig in einer Tinktur oder Creme vorliegen oder ein Wirkstoff liegt in einer Tinktur und der andere Wirkstoff liegt ebenfalls in einer Tinktur oder in einer Creme vor.

Die Kombination von Trockenextrakt des Passionsblumenkrauts und getrocknetem Miesmuschelfuß-Konzentrat, entweder in Form einer Festdosiskombination oder freien Dosiskombination, sowohl zur oralen als auch topischen Verabreichung, zeigt beim Krankheitsbild der Neurodermitis eine deutliche Beruhigung der entzündeten Haut, eine Stärkung der Festigkeit und reduzierte Schuppung der Haut sowie eine Glättung der Haut, wobei eine vorhandene Rötung abnimmt und der Juckreiz deutlich reduziert wird. Zudem wird ein besonders positiver Einfluss auf die Hautelastizität, die Hautfestigkeit, den Wasserhaushalt der Haut sowie die Zelldifferenzierung und -proliferation beobachtet. Es kann eine deutlich verbesserte Elastizität und eine signifikant verbesserte Festigkeit der Haut erreicht werden, wobei gleichzeitig der Wasserverlust des subkutanen Hautgewebes stark verringert wird. Insgesamt wird ein deutlich verbessertes Hautbild beobachtet. Es scheint, dass das Zusammenwirken der beiden Wirkstoffe, d.h. des Trockenextrakt des Passionsblumenkrauts und des getrockneten Miesmuschelfuß-Konzentrats, zu einem noch besseren Behandlungserfolg für Neurodermitis führt als beide Wirkstoffe allein, d.h. eine synergistische Wirkung wird angenommen.

Die pharmazeutische Zusammensetzung kann oral oder topisch verabreicht werden, mit der beschriebenen Einschränkung, dass der Trockenextrakt der Passionsblume nicht als alleiniger Wirkstoff topisch verabreicht wird.

Unter "topischer" Verabreichung wird hier im Unterschied zur sogenannten systemischen Verabreichung eines Arzneimittels die lokale Therapie verstanden, bei der der Wirkstoff oder die Wirkstoffe dort angewendet werden, wo diese therapeutisch wirken sollen. Im vorliegenden Fall ist die topische Verabreichung die örtliche und äußerliche Anwendung auf der Haut. Dies kann mit jeder dem Fachmann für diesen Zweck bekannten Dosierungsform erfolgen. Derartige Formulierungen werden in einer flüssigen, halbfesten oder festen Dosierungsform bereitgestellt und können beispielsweise in Form einer Lösung, insbesondere als Tinktur in Form von Tropfen, Öl, Suspension, insbesondere einer Schüttelmixtur, Emulsion, Lotion oder Milch, Creme oder Paste oder dergleichen vorliegen. Bevorzugte topische Verabreichungsformen sind Tinkturen, insbesondere in Form von Tropfen, oder eine Creme.

Die pharmazeutische Zusammensetzung, umfassend den Trockenextrakt des Passionsblumenkrauts und das getrocknete Miesmuschelfuß-Konzentrat oder nur das getrockneten Miesmuschelfuß-Konzentrat, kann daher in der gewählten Darreichungsform bzw. Formulierung auf die Haut lokal (topisch) aufgetragen werden.

Unter Tinktur zur topischen Verabreichung wird hier eine wässrige oder alkoholischwässrige Mischung mit dem getrockneten Miesmuschelfuß-Konzentrat und gegebenenfalls dem Trockenextrakt der Passionsblumenkrauts verstanden. Lösungsmittel sind Wasser und Wasser/Alkohol-Gemische. Es wird in der Regel eine Lösung gebildet, wobei - vor der Verabreichung - nicht gelöste Bestandteile durch Filtrieren entfernt werden können. Unter "Lösung" wird hier keine homogene, klare Flüssigkeit verstanden, sondern jede Form einer hauptsächlich flüssigen Mischung; diese umfasst auch eine Dispersion oder Suspension.

Fertige Tinkturen können auch käuflich erworben werden und sind in verschiedenen Konzentrationen erhältlich. Für die topische Verabreichung eignet sich beispielweise die Tinktur Ceres Passiflora Incarnata Urtinktur von der Firma CERES Heilmittel GmbH oder die Tinktur Naturalma Passionsblume (Passiflora incarnata), Kraut mit Blüten, alkoholfreier Urtinktur, von Naturalma srl, Bologna.

Ein anderer Verabreichungsweg ist die orale Verabreichung. Dies stellt eine systemische Verabreichung dar, da der gesamte Körper der Therapie unterzogen wird. Geeignete Zubereitungen zur oralen Verabreichung des Wirkstoffs/der Wirkstoffe gemäß der vorliegenden Erfindung umfassen beispielsweise Tabletten, insbesondere Filmtabletten, Pillen, Dragees, Pastillen, Pellets, Kapseln, insbesondere Hart- oder Weichkapseln, Pulver, Granulate, Lösungen, insbesondere Tinkturen in Form von Tropfen, Suspensionen, Dispersionen oder Emulsionen. Bevorzugt sind Filmtabletten, Kapseln, Tinkturen, insbesondere Tropfen. Die pharmazeutische Zusammensetzung, umfassend den Trockenextrakt des Passionsblumenkrauts und das getrocknete Miesmuschelfuß-Konzentrat oder nur das getrockneten Miesmuschelfuß-Konzentrat oder nur den Trockenextrakt des Passionsblumenkrauts, kann daher in der gewählten Darreichungsform bzw. Formulierung oral verabreicht werden.

Unter Tinktur zur oralen Verabreichung wird hier eine wässrige oder alkoholischwässrige Mischung mit dem Trockenextrakt der Passionsblumenkrauts und/oder getrocknetem Miesmuschelfuß-Konzentrat verstanden. Die obigen Ausführungen zur topischen Tinktur gelten entsprechend.

Es kann auch vorteilhaft sein, wenn zwei verschiedene orale Dosierungsformen miteinander kombiniert werden. Beispielweise kann zusätzlich zu einer Kapsel oder Tablette, die z.B. ein- bis dreimal täglich verabreicht wird, eine Tinktur, beispielweise ein- bis dreimal täglich verabreicht werden. Hierdurch kann die Dosierungsmenge in einfacher Weise für einen bestimmten Patienten zusätzlich variiert werden.

Zur therapeutischen Anwendung der pharmazeutischen Zusammensetzung kann irgendeine herkömmliche Dosierungsform, die für die orale oder topische Verabreichung geeignet ist, eingesetzt werden. Der bevorzugte Verabreichungsweg hängt u.a. vom Schweregrad der Neurodermitis und dem Zustand des Patienten ab.

Die Verabreichung des/der Wirkstoff(e)s der Erfindung erfolgt in einer geeigneten pharmazeutischen Zusammensetzung, in der nur der/die Wirkstoff(e) vorliegen oder in der der/die Wirkstoff(e) zusammen mit herkömmlichen pharmazeutisch akzeptablen Hilfsstoffen vorhanden sind. In der pharmazeutischen Zusammensetzung der vorliegenden Erfindung sind die einzigen Wirkstoffe Passionsblumen-Trockenextrakt und/oder Miesmuschelfuß-Konzentrat, weitere Wirksubstanzen sind in der pharmazeutischen Zusammensetzung nicht enthalten. Neben den Wirkstoffen in Form von Passionsblumen-Trockenextrakt und/oder Miesmuschelfuß-Konzentrat, können nur noch gegebenenfalls ein oder mehrere Hilfsstoffe in der pharmazeutischen Zusammensetzung vorhanden sein.

Pharmazeutisch akzeptable Hilfsstoffe sind beispielweise Trägerstoffe, übliche Zusätze, Verdünnungsmittel, Bindemittel, Sprengmittel und dergleichen sowie Kombinationen dieser. Der Begriff "pharmazeutisch akzeptabel" bedeutet im Rahmen der vorliegenden Erfindung Hilfsstoffe, welche für die beschriebene Behandlung eingesetzt werden können und aus medizinischer Sicht nicht unerwünscht sind, im Rahmen einer medizinischen Verabreichung an einen Verwender keinen nachteiligen und/oder schädlichen Einfluss zeigen, beispielsweise keine Reizungen, Allergien oder andere Probleme beim Verwender in Anbetracht der Schwere der Beeinträchtigung oder Erkrankung und der Notwendigkeit der Behandlung auslösen.

Derartige pharmazeutisch akzeptable bzw. annehmbare Hilfsstoffe genauso wie Verfahren zur Herstellung von pharmazeutischen Zusammensetzungen für die verschiedenen Verabreichungswege sind dem Fachmann im Stand der Technik gut bekannt.

Geeignete Tabletten können beispielsweise durch Mischen des oder der Wirkstoffe mit bekannten Hilfsstoffen, beispielsweise ein oder mehreren Verdünnungsmitteln, ein oder mehreren Bindemitteln, ein oder mehreren Gleit- oder Schmiermitteln, optional ein oder mehreren Sprengmitteln oder Zerfallhilfsmitteln sowie optional weiteren Zusätzen, hergestellt werden. Einige der Hilfsstoffe können zwei oder mehr Funktionen gleichzeitig aufweisen, z.B. als ein Bindemittel, Verdünnungsmittel und Füllstoff wirken.

Jegliches Verdünnungsmittel, das in Tabletten herkömmlicherweise eingesetzt wird, kann Verwendung finden. Geeignete Verdünnungsmittel sind beispielsweise Lactose, insbesondere Lacosemonohydrat, Cellulose und Derivate, wie Cellulosepulver, Stärken und deren Derivate, Glucose, Oligosaccharide, wie Saccharose, Maltodextrin oder Calciumcarbonat. Bevorzugte Verdünnungsmittel sind Lactosemonohydrat und Cellulosepulver.

Jedes Gleit- oder Schmiermittel, das in Tabletten üblicherweise eingesetzt wird, kann verwendet werden. Geeignete Gleitmittel sind beispielweise Siliciumdioxid, insbesondere hochdisperses Siliciumdioxid, Talk, Natriumdodecylsulfat, Stearinsäure und deren Salze, wie Magnesiumstearat. Bevorzugt ist das Magnesiumstearat ein pflanzliches Produkt, wie beispielsweise ein Magnesiumsalz von aus Sonnenblumenöl gewonnenen Fettsäuren.

Als Sprengmittel kommt jedes, das in Tabletten üblicherweise eingesetzt wird, in Frage. Ein geeignetes Sprengmittel ist beispielweise Croscarmellose-Natrium oder Maisstärke.

Jedes bekannte Bindemittel, das in Tabletten in der Regel eingesetzt wird, kann eingesetzt werden. Bindemittel sind beispielsweise natürlich auftretende oder teilweise oder vollständig synthetische Polymere, ausgewählt aus Cellulose und Derivaten hiervon, z.B. Hypromellosen (Cellulosehydroxypropylmethylether), Stärke und Derivaten hiervon, Tragant und Gelatine. Die Gelatine ist bevorzugt kein tierisches Produkt und stammt aus pflanzlichen Quellen, beispielweise Rotalgen. Bevorzugt wird daher vegane Gelatine eingesetzt.

Optional können in den Tabletten auch färbende Zusatzstoffe, wie beispielsweise Titandioxid (Lebensmittelzusatzstoffe E 171), kupferhaltige Komplexe der Chlorophylle und Chlorophylline (Lebensmittelfarbstoff E 141), bevorzugt aus pflanzlichem Ursprung, oder Eisenoxid-Pigmente zum Einsatz kommen.

Die Tabletten können beschichtet sein und eine Schicht, wie beispielweise in einer Filmtablette, oder auch mehrere Schichten aufweisen. Beschichtungen sind beispielsweise ausgewählt aus Kollidon, Schellack, Wachse, wie Carnaubawachs, Gummi arabicum, Talk, Titandioxid, Zucker, Hydroxypropylmethylcellulose sowie Mischungen dieser und können auch zusätzliche Hilfsstoffe enthalten. Eine Filmbeschichtung umfasst beispielweise ein Filmbildungsmittel, einen Weichmacher, ein Gleitmittel und gegebenenfalls ein oder mehrere Pigmente. Eine exemplarische Filmbeschichtungszusammensetzung kann umfassen: Hydroxypropylmethylcellulose (HPMC), Polyethylenglykol (PEG), Talk, Titandioxid und gegebenenfalls Eisenoxid, einschließlich Eisenoxid rot und/oder gelb.

Kapseln, enthaltend den oder die Wirkstoffe können beispielsweise durch Mischen des oder der Wirkstoffe mit geeigneten pharmazeutisch akzeptablen Hilfsstoffen und Verpacken dieser in Hart- oder Weichkapseln, hergestellt werden. Die geeigneten pharmazeutisch akzeptablen Hilfsstoffe sind dem Fachmann bekannt und entsprechen im Wesentlichen den pharmazeutisch akzeptablen Hilfsstoffen, die im Zusammenhang mit Tabletten im Einzelnen beschrieben wurden.

Weitere pharmazeutisch akzeptable Hilfsstoffe, die insbesondere für flüssige Zubereitungen verwendet werden, sind beispielweise Pflanzenöle und -wachse, wie Jojobalöl, Olivenöl, Mandelöl, Sheabutter; ätherische Öle; Emulgatoren, insbesondere Emulgatoren aus pflanzlichem Ursprung, wie Temulgator (ein pflanzlicher Emulgator aus Palmöl); Vitamine, wie z.B. D-alpha-Tocopherylsuccinat (Vitamin E); Lösungsmittel, wie destilliertes Wasser, Alkohol, wie Ethanol, Glycerin, Phenethylalkohol und dergleichen. Andere pharmazeutisch akzeptable Hilfsstoffe, die hier nicht explizit genannt sind, aber dem Fachmann im Stand der Technik bekannt sind, können ebenfalls eingesetzt werden.

Flüssige Zubereitungen, wie Lösungen, insbesondere Tinkturen, oder dergleichen, werden in üblicher Art und Weise hergestellt, z.B. unter Zugabe von Lösungsmitteln, insbesondere Wasser für pharmazeutische Zwecke, d.h. destilliertes Wasser, oder Wasser/Alkohol-Gemischen zu dem oder den Wirkstoffen, insbesondere dem Trockenextrakt von Passionsblumenkraut und/oder getrocknetem Miesmuschelfuß-Konzentrat und optional anschließendem Filtrieren der Lösung. Verwendete Alkohole sind beispielsweise Ethanol oder Glycerin.

Gemäß einer weiteren Ausführungsform kann eine handelsübliche Tinktur als pharmazeutische Zusammensetzung verwendet werden, die optional mit destilliertem Wasser, verdünnt wird. Da nur für das Passionsblumenkraut eine Tinktur im Handel erhältlich ist, kann das getrocknete Miesmuschelfuß-Konzentrat auch optional zu der käuflich erworbenen Tinktur in geeigneter Menge zugesetzt werden, wenn ein Kombinationspräparat für eine orale oder topische Verabreichung hergestellt werden soll. Die Tinktur mit dem/den Wirkstoff(en) und dem Lösungsmittel kann unmittelbar als pharmazeutische Zusammensetzung eingesetzt werden.

In Einzelfällen kann es vorteilhaft sein, wenn die pharmazeutische Zusammensetzung frei von Aromen, Farbstoffen und Stabilisatoren, laktosefrei, glutenfrei und/oder frei von Konservierungsstoffen ist.

Wenn Hilfsstoffe von pflanzlichem und nicht-pflanzlichem Ursprung sind, kann es vorteilhaft sein, wenn Hilfsstoffe derart ausgewählt werden, dass diese von pflanzlichem Ursprung sind. Gemäß einer Ausführungsform kann es bevorzugt sein, wenn in der pharmazeutischen Zusammensetzung nur pflanzliche Hilfsstoffe eingesetzt werden.

Insbesondere kann es bevorzugt sein, wenn nur vegane Hilfsstoffe zum Einsatz kommen, d.h. von Tieren stammende Hilfsstoffe oder von Tierprodukten stammende oder abgeleitete Hilfsstoffe sind dann nicht enthalten. Dies kann im Hinblick auf mögliche Allergien von Vorteil sein.

Die Dosierung des oder der erfindungsgemäßen Wirkstoffe ist natürlich hochgradig abhängig vom gewählten Verabreichungsweg und dem Krankheitsbild der Neurodermitis, die behandelt werden soll.

Für die orale Verabreichung liegt die bevorzugte (Dosierungs-)Menge des Trockenextrakts des Passionsblumenkrauts, die in der pharmazeutischen Zusammensetzung vorhanden ist, pro fester oraler Dosierungsform (z.B. enthalten in einer Tablette oder einer Kapsel), im Bereich von 45 bis 500 mg oder 90 bis 450 mg oder 100 bis 450 mg oder 150 bis 450 mg oder 200 bis 450 mg oder 250 bis 450 mg. Bevorzugte (Dosierungs-)Mengen in der pharmazeutischen Zusammensetzung (z.B. pro Tablette oder Kapsel) sind daher beispielsweise 45 mg, 50 mg, 90 mg, 100 mg, 150 mg, 200 mg, 250 mg, 300 mg, 350 mg, 400 mg, 450 mg oder 500 mg, insbesondere 350 oder 400 mg, des Trockenextrakts des Passionsblumenkrauts. Die orale Verabreichung der pharmazeutischen Zusammensetzung kann beispielweise bis zu fünfmal am Tag erfolgen, d.h. ein- bis fünfmal täglich oder ein- bis viermal täglich oder ein- bis dreimal täglich oder ein- bis zweimal täglich oder einmal täglich. Die Gesamtdosis beträgt dann etwa 45 bis 2500 mg oder 90 bis 2250 mg oder 100 bis 2000 mg oder 225 bis 1800 mg oder 400 bis 1500 mg für einen Patienten pro Tag. Die Dosierung wird abhängig vom Körpergewicht des Patienten und dem Schweregrad der Neurodermitis ausgewählt. Dies hängt von jedem Einzelfall ab.

Für die orale Verabreichung liegt die bevorzugte (Dosierungs-)Menge des getrockneten Miesmuschelfuß-Konzentrats, die in der pharmazeutischen Zusammensetzung vorhanden ist, pro fester oraler Dosierungsform (z.B. enthalten in einer Tablette oder einer Kapsel), im Bereich von 1 mg bis 50 mg, 1 bis 45 mg, 1 bis 40 mg, 1 bis 35 mg, 1 bis 30 mg, 1 bis 25 mg, 1 bis 20 mg, 1 bis 15 mg oder 2 bis 12 mg oder 3 bis 10 mg. Bevorzugte (Dosierungs-)Mengen in der pharmazeutischen Zusammensetzung (z.B. pro Tablette oder Kapsel) sind daher beispielsweise 1 mg, 2 mg, 3, mg, 4 mg, 5 mg, 6 mg, 7 mg, 8, mg, 9 mg, 10 mg, 11 mg, 12 mg, 13 mg, 14 mg oder 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg oder 50 mg getrocknetes Miesmuschelfuß-Konzentrat. Die Gesamtdosis beträgt dann bspw. etwa 5 bis 250 mg oder 10 bis 200 mg oder 15 bis 150 mg oder 15 bis 100 mg für einen Patienten pro Tag.

In der pharmazeutischen Zusammensetzung zur oralen Verabreichung kann als Wirkstoff(e) der Trockenextrakt des Passionsblumenkrauts und/oder das getrocknete Miesmuschelfuß-Konzentrat vorliegen. Bevorzugt liegen in der pharmazeutischen Zusammensetzung zur oralen Verabreichung als Wirkstoffe der Trockenextrakt des Passionsblumenkrauts und das getrocknete Miesmuschelfuß-Konzentrat zusammen vor.

Gemäß einer Ausführungsform wird als Dosierungsform für die pharmazeutische Zusammensetzung zur oralen Verabreichung eine Kapsel ausgewählt. Die Kapsel weist als pharmazeutische Zusammensetzung folgende Wirkstoffe auf:
- Passionsblumenkraut in Form eines Trockenextrakts und optional Hilfsstoffe; oder
- Passionsblumenkraut in Form eines Trockenextrakts und getrocknetes Miesmuschelfuß-Konzentrat und optional Hilfsstoffe; oder
- getrocknetes Miesmuschelfuß-Konzentrat und optional Hilfsstoffe.

Der oder die Wirkstoffe können in der Kapsel an sich vorliegen, d.h. ohne Hilfsstoffe enthalten sein.

Die Kapsel umfasst bevorzugt folgende Bestandteile:

| | |
|---|---|
| - Trockenextrakt aus Passionsblumenkraut: (beispielweise ein 5:1 Extrakt) | 5,0 - 99,9 Gew.-% |

und

| | |
|---|---|
| - getrocknetes Miesmuschelfuß-Konzentrat: | 0,1 - 10,0 Gew.-%, |

oder

| | |
|---|---|
| - Trockenextrakt aus Passionsblumenkraut: | 5,0 - 100 Gew.-% |

oder

| | |
|---|---|
| - getrocknetes Miesmuschelfuß-Konzentrat: | 0,1 - 100 Gew.-% |

(für das getrocknete Miesmuschelfuß-Konzentrat bevorzugt ausgehend von Muscheln aus dem Naturschutzgebiet Wattenmeer vor Sylt)
und optional die folgenden Hilfsstoffe:

| | |
|---|---|
| - Lactose-Monohydrat | 0 - 45 Gew.-% |
| - Cellulosepulver | 0 - 45 Gew.-% |
| - hochdisperses Siliciumdioxid | 0 - 5,0 Gew.-% |
| - pflanzliches Magnesiumstearat | 0 - 5,0 Gew.-% |
| - vegane Gelatine aus Rotalgen | 0 - 5,0 Gew.-% |
| - Natriumdodecylsulfat | 0 - 5,0 Gew.-% |
| - Chlorophyllin-Kupfer-Komplex (E 141) | 0 - 2,0 Gew.-% |
| - Titandioxid (E171) | 0 - 5,0 Gew.-% |

wobei die Mengen der jeweiligen Komponenten so gewählt sind, dass das Gesamtgewicht der Komponenten 100 Gew.-% nicht übersteigt.

Eine weitere bevorzugte Dosierungsform für die pharmazeutische Zusammensetzung zur oralen Verabreichung ist eine Tablette, insbesondere eine Filmtablette. Die Tablette weist als pharmazeutische Zusammensetzung folgende Wirkstoffe auf:
- Passionsblumenkraut in Form eines Trockenextrakts und Hilfsstoffe; oder
- Passionsblumenkraut in Form eines Trockenextrakts und getrocknetes Miesmuschelfuß-Konzentrat sowie Hilfsstoffe; oder
- getrocknetes Miesmuschelfuß-Konzentrat und Hilfsstoffe.

Gemäß einer Ausführungsform weist eine Tablette folgende Bestandteile auf:

| | |
|---|---|
| - Trockenextrakt aus Passionsblumenkraut: bevorzugt 9,0 - 93,4 Gew.-%, (beispielweise ein 5:1 Extrakt) | 5,0 - 93,4 Gew.-%, |

und

| | |
|---|---|
| - getrocknetes Miesmuschelfuß-Konzentrat: | 0,1 - 10 Gew.-%, |

oder

| | |
|---|---|
| - Trockenextrakt aus Passionsblumenkraut: | 5,0 - 93,5 Gew.-% |

oder

| | |
|---|---|
| - getrocknetes Miesmuschelfuß-Konzentrat: | 0,1 - 93,5 Gew.-% |

(für das getrocknete Miesmuschelfuß-Konzentrat bevorzugt ausgehend von Muscheln aus dem Naturschutzgebiet Wattenmeer vor Sylt)
und die folgenden Hilfsstoffe:

| | |
|---|---|
| - Lactose-Monohydrat | 2,5 - 45,0 Gew.-% |
| - Cellulosepulver | 3,5 - 45,0 Gew.-% |
| - hochdisperses Siliciumdioxid | 0 - 5,0 Gew.-% |
| - pflanzliches Magnesiumstearat | 0,5 - 5,0 Gew.-% |
| - vegane Gelatine aus Rotalgen | 0 - 5,0 Gew.-% |
| - Natriumdodecylsulfat | 0 - 5,0 Gew.-% |
| - Chlorophyllin-Kupfer-Komplex (E 141) | 0 - 2,0 Gew.-% |
| - Titandioxid (E171) | 0 - 5,0 Gew.-%, |

wobei die Mengen der jeweiligen Komponenten so gewählt sind, dass das Gesamtgewicht der Komponenten 100 Gew.-% nicht übersteigt.

Die Tablette kann mit einer zusätzlichen Schicht versehen sein, die beispielweise aus Kollidon, Schellack, Gummi arabicum, Talk, Titandioxid, Zucker und Hydroxypropylmethylcellulose oder Mischungen dieser aufgebaut ist.

Wenn für die orale Verabreichung eine flüssige Dosierungsform verwendet wird, wie eine Tinktur, beispielsweise in Form von Tropfen, kann in der pharmazeutischen Zusammensetzung eine geeignete Menge an Trockenextrakt des Passionsblumenkraut und/oder Miesmuschelfuß-Konzentrat eingesetzt werden, die je nach dem eingesetzten Trockenextrakt des Passionsblumenkrauts sehr unterschiedlich sein kann:
Für eine orale flüssige Darreichungsform, beispielweise eine Tinktur, liegt die Dosierungsmenge im oben angegebenen Bereich. Um eine flüssige Dosierungsform herzustellen werden daher etwa 10 bis 100 g des Trockenextrakts des Passionsblumenkrauts eingesetzt. Bevorzugte Mengen sind beispielsweise 10 g, 15 g, 20 g, 25 g, 30 g, 35 g, 40 g, 45 g, 50 g, 55 g, 60 g, 65 g, 70 g, 75 g, 80 g, 85 g, 90 g, 95 g oder 100 g des Trockenextrakts des Passionsblumenkrauts.

Beispielsweise kann eine Tinktur zur oralen Verabreichung, ausgehend von 10 g Trockenextrakt von Passionsblumenkraut (beispielweise ein 5:1 Extrakt), aufgefüllt mit einem Lösungsmittel aus einer Alkohol/Wasser-Mischung auf 200 g, hergestellt werden. Das Lösungsmittelgemisch kann beispielweise in einem Verhältnis von 1 (Alkohol): 1 bis 10 (destilliertes Wasser) vorliegen. Der Alkohol ist beispielweise Ethanol oder Glycerin. Die Herstellung wird bei den Beispielen im Einzelnen erläutert. Die erhaltene Tinktur kann beispielweise 1-mal täglich mit beispielweise 20 Tropfen (20 Tropfen sind etwa 1 ml, 1 ml enthält bei 10 g Trockenextrakt 50 mg Wirkstoff) auf einem Löffel oder weiter mit Wasser verdünnt eingenommen werden.

Es kann auch eine Tinktur, ausgehend von 100 g Trockenextrakt von Passionsblumenkraut (beispielweise: ein 5:1 Extrakt), mit einem Lösungsmittel auf 200g Lösung aufgefüllt, hergestellt werden. Das Lösungsmittel ist destilliertes Wasser oder eine Mischung aus Alkohol und destilliertem Wasser. Die erhaltene Tinktur kann beispielweise 1-mal täglich mit beispielweise 20 Tropfen (20 Tropfen sind etwa 1 ml, 1 ml enthält 500 mg Wirkstoff) auf einem Löffel oder weiter mit Wasser verdünnt eingenommen werden. Die Tinktur kann ein- bis fünfmal täglich, in Einzelfällen sogar sechsmal täglich, oder ein- bis viermal täglich oder ein- bis dreimal täglich oder einbis zweimal täglich oder auch nur einmal täglich eingenommen werden, abhängig vom Krankheitsbild der Neurodermitis. Dies hängt von jedem Einzelfall ab. Beispielsweise kann auch eine Tinktur speziell für einen Patienten mit der geeigneten Dosierungsstärke hergestellt werden. Die Tinktur kann auch käuflich erworben werden; beispielweise kommt die Tinktur Ceres Passiflora Incarnata Urtinktur von der Firma CERES Heilmittel GmbH oder die Tinktur Naturalma Passionsblume (Passiflora incarnata) Kraut mit Blüten, alkoholfreier Urtinktur von Naturalma srl, Bologna, in Frage.

Für die orale Verabreichung in Form einer flüssigen Dosierungsform, beispielweise als Tinktur, wird zu dessen Herstellung bevorzugt eine Menge des getrockneten Miesmuschelfuß-Konzentrats im Bereich von 0,20 bis 6,0 g eingesetzt, die beispielweise mit einem Lösungsmittel auf 200 g Lösung aufgefüllt wird. Das Lösungsmittel ist destilliertes Wasser oder eine Mischung aus Alkohol und destilliertem Wasser. Die erhaltene Tinktur kann beispielweise 1-mal täglich mit beispielweise 20 Tropfen (20 Tropfen sind dann etwa 1 ml, 1 ml enthält 1 - 30 mg Wirkstoff) verabreicht werden.

So kann eine Tinktur zur oralen Verabreichung bevorzugt umfassen:
10 - 100 g Trockenextrakt von Passionsblumenkraut (5 - 50 Gew.-%ige-Lösung, ergibt etwa 50 bis 500 mg in 20 Tropfen der Tinktur) und/oder
0,20 bis 6,0 g Miesmuschelfuß-Konzentrat (0,1 - 3,0 Gew.-%ige Lösung, ergibt etwa 1 bis 30 mg in 20 Tropfen der Tinktur),
mit einem Lösungsmittel auf 200 g Lösung aufgefüllt, wobei das Lösungsmittel ausgewählt ist aus Wasser oder einer Alkohol/Wasser-Mischung mit einem Verhältnis von 1 : 1 bis 10.

Wenn der Trockenextrakt des Passionsblumenkrauts und das getrocknete Miesmuschelfuß-Konzentrat in der Tinktur zusammen vorliegen sollen, werden die Mengen beider Wirkstoffe vorgelegt und mit Lösungsmittel, wie bereits beschrieben, aufgefüllt.

Für eine Tinktur für die topische Verabreichung liegen die bevorzugten (Dosierungs-)Mengen des getrockneten Miesmuschelfuß-Konzentrats und des gegebenenfalls vorhandenen Trockenextrakts des Passionsblumenkrauts in denselben Bereichen wie für die Tinktur zur oralen Verabreichung. Für die Tinktur zur topischen Verabreichung kann es zweckmäßig sein, wenn eine höhere (Dosierungs-)Menge verwendet wird (z.B. 27,5 - 50 Gew.-%ige Lösung für den Trockenextrakt der Passionsblumenkrauts und 1,5 - 3,0 Gew.-%ige Lösung für das getrocknete Miesmuschelfuß-Konzentrat), wohingegen für eine Tinktur zur oralen Verabreichung die (Dosierungs-)Mengen niedriger liegen können (z.B. 5 - 27,5 Gew.%ige Lösung für den Trockenextrakt der Passionsblumenkrauts und 0,1 - 1,5 Gew.-%ige Lösung für das getrocknete Miesmuschelfuß-Konzentrat).

Es ist hierbei zu berücksichtigen, dass sich die Trockenextrakte des Passionsblumenkrauts hinsichtlich ihrer Dosierungsstärke unterscheiden können, da die Konzentration der Extrakte, je nach der Menge und der Art des Passionsblumenkraut-Ausgangsmaterials, variieren kann. Übliche Konzentrationen sind, wie bereits erläutert, Trockenextrakte von 5:1 bis 7:1, wobei auch 10:1 vorliegen kann. Dies wird dann dadurch berücksichtigt, dass entweder die Menge in der Dosierungsform entsprechend angepasst wird und/oder die Verabreichungshäufigkeit herauf oder herabgesetzt wird, beispielweise von 5mal täglich auf 3mal täglich für einen höher konzentrierten Extrakt, der in der pharmazeutischen Zusammensetzung vorliegt. Die angegebenen Mengen gehen hier in der Regel vom 5:1 Extrakt aus, sofern nichts anderes angegeben ist.

Erfindungsgemäß ist neben dem Trockenextrakt des Passionsblumenkrauts bei topischer Verabreichung stets ein Miesmuschelfuß-Konzentrat vorhanden.

Somit kann eine Tinktur zur topischen Verabreichung bevorzugt umfassen:
0,20 bis 6,0 g Miesmuschelfuß-Konzentrat (0,1 - 3,0 Gew.-%ige Lösung, ergibt etwa 1 bis 30 mg in 20 Tropfen der Tinktur),
optional 10 - 100 g Trockenextrakt von Passionsblumenkraut (5 - 50 Gew.-%ige-Lösung, ergibt etwa 50 bis 500 mg in 20 Tropfen der Tinktur)
mit einem Lösungsmittel auf 200 g Lösung aufgefüllt, wobei das Lösungsmittel ausgewählt ist aus Wasser oder einer Alkohol/Wasser-Mischung mit einem Verhältnis von 1 : 1 bis 10.

Eine weitere bevorzugte Dosierungsform für die topische Verabreichung ist eine Creme. Gemäß einer Ausführungsform weist die Creme folgende Bestandteile auf:

| | |
|---|---|
| getrocknetes Miesmuschelfuß-Konzentrat | 0,5 - 10,00 Gew.-% |

(besonders bevorzugt ausgehend von Muscheln aus dem Naturschutzgebiet Wattenmeer vor Sylt) und

| | |
|---|---|
| optional Trockenextrakt von Passionsblumenkraut sowie | 0 - 50,00 Gew.-% |
| Jojobaöl | 5,00 - 25,00 Gew.-% |
| Mandelöl | 1,00 - 15,00 Gew.-% |
| Olivenöl | 1,00 - 8,00 Gew.-% |
| Temulgator (pflanzlicher Emulgator aus Palmöl) | 1,00 - 10,00 Gew.-% |
| Sheabutter (bevorzugt raffiniert) | 1,00 - 10,00 Gew.-% |
| D-alpha-Tocopherylsuccinat (Vitamin E) | 0,10 - 2,00 Gew.-% |
| Phenetylalkohol | 0,10 - 1,00 Gew.-% |
| ätherische(s) Öl(e) | 0,10 - 2,00 Gew.-% |
| destilliertes Wasser | auf 100 Gew.-%. |

Die topische Verabreichung der pharmazeutischen Zusammensetzung kann ein- bis fünfmal täglich, in Einzelfällen auch häufiger, oder ein- bis viermal täglich oder ein- bis dreimal, ein- bis zweimal täglich oder nur einmal täglich erfolgen. Die Dosierung wird abhängig vom Körpergewicht des Patienten und dem Schweregrad der Neurodermitis ausgewählt. Dies hängt von jedem Einzelfall ab. Beispielsweise kann auch eine Darreichungsform speziell für einen Patienten mit der geeigneten Dosierungsstärke hergestellt werden.

Die Gesamtdosis der einzelnen Wirkstoffe wird abhängig von der Dosierungsform, dem Körpergewicht des Patienten und der Ausprägung der Neurodermitis ausgewählt. Dies hängt vom Einzelfall ab. Wenn ein Kombinationspräparat zum Einsatz kommt, können die einzelnen Dosismengen zum Teil reduziert werden.

Die Vorteile der vorliegenden Erfindung sind außerordentlich vielschichtig:
Die erfindungsgemäße pharmazeutische Zusammensetzung findet im therapeutischen Bereich zur Behandlung von Neurodermitis Verwendung. Die Verabreichung erfolgt oral oder durch Auftragung auf die Haut (topisch) und ist hinsichtlich der gewählten Verabreichungsform nicht besonders beschränkt, sofern die Einschränkung gemäß der vorliegenden Erfindung berücksichtigt wird. Der Verabreichungsweg ist für eine pharmazeutische Zusammensetzung, umfassend einen Trockenextrakt des Passionsblumenkrauts bevorzugt oral, für eine Kombination von Trockenextrakt des Passionsblumenkrauts und getrocknetes Miesmuschelfuß-Konzentrat ebenfalls bevorzugt oral und für getrocknetes Miesmuschelfuß-Konzentrat bevorzugt topisch.

In völlig unerwarteter Weise konnte festgestellt werden, dass die erfindungsgemäßen Wirkstoffe, jeweils oder in Kombination, zu besonders guten Behandlungserfolgen bei Neurodermitis führen. Dabei wird eine Verminderung der Hautrötung, deutliche Reduzierung des Juckreizes, Stärkung der Festigkeit der Haut, Verminderte Schuppung der Haut, Glättung der Haut und insgesamt eine Beruhigung der entzündeten Haut festgestellt. Insgesamt wird durch die Behandlung ein besseres Hautbild erhalten. Die pharmazeutische Zusammensetzung wirkt sich insgesamt daher positiv auf die Haut und das Hautbild aus.

Die nachteiligen Wirkungen, die mit gängigen Arzneimitteln, wie beispielsweise Glukokortikoiden, auftreten, wie Ausdünnen der Haut, Hautveränderungen oder zusätzliche Reizungen, können vollständig vermieden werden.

Insbesondere durch die Kombinationswirkung von Trockenextrakt aus Passionsblumenkraut und getrocknetem Miesmuschelfuß-Konzentrat können besonders gute Behandlungsergebnisse erzielt werden. Die durch die Neurodermitis verursachten Entzündungen klingen innerhalb kurzer Zeit weitgehend ab.

Durch die deutliche Verbesserung des Hautbildes kann auf eine zusätzliche Verabreichung von klassischen Arzneimitteln verzichtet werden.

Die positive Wirkung der Wirkstoffe auf die Haut steht insgesamt im Vordergrund, wobei die natürliche Mikroflora der Haut nicht geschädigt, sondern vielmehr intakt erhalten bleibt. So steigt die Hautelastizität, die Hautfestigkeit nimmt zu, der Wasserhaushalt der Haut sowie die Zelldifferenzierung und -proliferation werden nachhaltig positiv beeinflusst. Außerdem wird das Erscheinungsbild der Haut deutlich verbessert.

Im nachfolgenden experimentellen Teil sind einige Versuche detailliert geschildert, in denen die Herstellung und die therapeutische Wirksamkeit der erfindungsgemäßen pharmazeutischen Zusammensetzung beschrieben sind. Selbstverständlich soll die vorliegende Erfindung hierdurch nicht beschränkt werden.

### Beispiele

### Herstellung einiger beispielhafter pharmazeutischer Dosierungsformen

### Kapseln

Die verwendeten Bestandteile werden nachfolgend angegeben:
Wirkstoffe:

| | |
|---|---|
| - Trockenextrakt aus Passionsblumenkraut: (5:1 Extrakt) und | 400 mg |
| - getrocknetes Miesmuschelfuß-Konzentrat: (ausgehend von Muscheln aus dem NaturschutzgebietWattenmeer vor Sylt) | 5mg |

Hilfsstoffe:

| | |
|---|---|
| - Lactose-Monohydrat | 5,0 mg |
| - Cellulosepulver | 5,0 mg |
| - hochdisperses Siliciumdioxid | 5,0 mg |
| - pflanzliches Magnesiumstearat | 5,0 mg |

| | |
|---|---|
| - vegane Gelatine aus Rotalgen | 5,0 mg |
| - Natriumdodecylsulfat | 5,0 mg |
| - Chlorophyllin-Kupfer-Komplex (E 141) | 5,0 mg |
| - Titandioxid (E171) | 5,0 mg |

Herstellung:
Die Wirkstoffe werden mit den Hilfsstoffen zusammengemischt und gegebenenfalls gesiebt. Die fertig gestellte Mischung wird in Hartkapseln der Größe 0 (mögliche Füllmenge 400 - 800 mg) gepackt.

| | |
|---|---|
| Kapselfüllung: | 445 mg |

### Tabletten

Die verwendeten Bestandteile werden nachfolgend angegeben: Wirkstoffe:

| | |
|---|---|
| - Trockenextrakt aus Passionsblumenkraut: (5:1 Extrakt) und | 400 mg |
| - getrocknetes Miesmuschelfuß-Konzentrat: (ausgehend von Muscheln aus dem NaturschutzgebietWattenmeer vor Sylt) | 5 mg |

Hilfsstoffe:

| | |
|---|---|
| - Lactose-Monohydrat | 15,0 mg |
| - Cellulosepulver | 20,0 mg |
| - hochdisperses Siliciumdioxid | 5,0 mg |
| - pflanzliches Magnesiumstearat | 5,0 mg |
| - vegane Gelatine aus Rotalgen | 5,0 mg |
| - Natriumdodecylsulfat | 5,0 mg |
| - Chlorophyllin-Kupfer-Komplex (E 141) | 5,0 mg |
| - Titandioxid (E171) | 5,0 mg |

Herstellung:
Die Wirkstoffe werden mit sämtlichen Komponenten gemischt, gesiebt und in einer Tabletten-Herstellungsvorrichtung komprimiert, um Tabletten der gewünschten Form zu bilden.

| | |
|---|---|
| Gewicht des Tablettenkerns: | 470 mg |
| Erscheinungsbild des Tablettenkerns: | 10 mm, rund |

Die derart hergestellten Tablettenkerne werden mit einem Film, bestehend im Wesentlichen aus Hydroxypropylmethylcellulose, beschichtet.

| | |
|---|---|
| Gewicht der beschichteten Tablette: | etwa 520 mg |

### Tinktur

Am Beispiel einer Tinktur zur oralen Verabreichung aus dem Trockenextrakt des Passionsblumenkrauts:
Herstellung:
10 g Trockenextrakt aus Passionsblumenkraut (5:1 Extrakt) werden in einem verschließbaren Behälter mit einer Glycerin/Wasser-Mischung (1:1) auf 200 g aufgegossen. Der Behälter wird verschlossen und mindestens vier Wochen an einem kühlen, dunklen Ort stehen gelassen. Danach wird in eine sterile, dunkel getönte Flasche filtriert. Die erhaltene Tinktur kann direkt oder mit Wasser verdünnt oral verabreicht werden.

### Creme

Am Beispiel einer Creme mit dem getrockneten Miesmuschelfuß-Konzentrat:
Es wurde eine Creme aus folgenden Bestandteilen hergestellt:

| | |
|---|---|
| getrocknetes Miesmuschelfuß-Konzentrat | 1,13 Gew.-% |
| (ausgehend von Muscheln aus dem Naturschutzgebiet Wattenmeer vor Sylt) | |
| Jojobaöl | 15,05 Gew.-% |
| Mandelöl | 11,29 Gew.-% |
| Olivenöl | 3,76 Gew.-% |
| Temulgator (pflanzlicher Emulgator aus Palmöl) | 5,65 Gew.-% |
| raffinierte Sheabutter | 5,65 Gew.-% |
| D-alpha-Tocopherylsuccinat (Vitamin E) | 0,23 Gew.-% |
| Phenetylalkohol | 0,30 Gew.-% |
| ätherisches Öl Meeresbrise | 0,34 Gew.-% |
| ätherisches Öl | 0,15 Gew.-% |
| destilliertes Wasser | auf 100 Gew.-% |

### Versuche

Es wurde seit März 2018 eine Reihe an Versuchen zur Behandlung von Neurodermitis durchgeführt, die noch andauern.

Es werden Tabletten und Tinkturen (Tropfen) mit verschiedenen Konzentrationen der Wirkstoffe jeweils für die orale Verabreichung getestet.

Es werden folgende Tabletten aus dem Handel eingesetzt:
- LIORAN centra überzogene Tabletten von der Firma Niehaus Pharma GmbH & Co. KG:
   Wirkstoff: Passionsblumen-Trockenextrakt 425 mg pro 1 Tablette (Extrakt 5-7:1); Auszugsmittel: Ethanol 50% (Vol./Vol.). Weitere Bestandteile der Tablette gemäß den Herstellerangaben sind: Maltodextrin, Siliciumdioxid, hochdisperses Cellulosepulver, Croscarmellose-Natrium, Magnesiumstearat (pflanzlich), Stearinsäure (pflanzlich), Talkum, Saccharose, Calciumcarbonat, Gummi arabicum, Tragant, Glucose-Sirup, sprühgetrocknete Glucose, Hypromellose, Wachs, gebleichtes Carnaubawachs, Schellack, Titandioxid und Eisen(III)-oxidhydrat, gelb.
- PASSIO Balance überzogene Tabletten (Filmtabletten) von der Firma STADA Consumer Health Deutschland GmbH:
   Wirkstoff: Passionsblumen-Trockenextrakt 425 mg pro 1 Tablette (Extrakt 5-7:1); Auszugsmittel: Ethanol 50% (V/V). Weitere Bestandteile der Tablette gemäß den Herstellerangaben sind: Gummi arabicum, Calciumcarbonat, Carnaubawachs, Cellulosepulver, Croscarmellose-Natrium, Glucose-Sirup, sprühgetrocknete Hypromellose, Magnesiumstearat (pflanzlich), Maltodextrin, Schellack, Stearinsäure, Saccharose, Talkum, Tragant, Wachs, gebleichtes Siliciumdioxid, hochdisperses Eisen(III)-oxidhydrat, gelb und Titandioxid.
- PASCOFLAIR überzogene Tabletten (Filmtabletten) der Firma Pascoe pharmazeutische Präparate GmbH:
   Wirkstoff: Passionsblumen-Trockenextrakt 425 mg pro 1 Tablette (Extrakt 5-7:1); Auszugsmittel: Ethanol 50% (V/V). Weitere Bestandteile der Tablette gemäß den Herstellerangaben sind: Maltodextrin, Siliciumdioxid, hochdisperses Cellulosepulver, Croscarmellose-Natrium, Magnesiumstearat (pflanzlich), Stearinsäure, Talkum, Saccharose, Calciumcarbonat, Gummi arabicum, Tragant, Glucosesirup-Trockensubstanz, Hypromellose, Wachs, gebleichtes Carnaubawachs, Schellack und Eisen(III)-oxidhydrat, gelb.

Von den obigen Tabletten wurde jeweils ein Handelsprodukt ausgewählt und hiervon 3 Tabletten täglich an einen Patienten verabreicht.

Alternativ wurde eine Tinktur verabreicht.

Es wird folgende Tinktur aus dem Handel eingesetzt:
- Naturalma Passionsblume (Passiflora incarnata), Kraut mit Blüten, Alkoholfreie Urtinktur von der Firma Naturalma srl, Bologna.

Nach den Herstellerangaben sind die Bestandteile: pflanzliches Glycerin, reines Wasser, Passionsblume (Passiflora incarnata), Kraut mit Blüten; konzentriert 1:10; 40 Tropfen (2 ml) enthalten 200 mg Passionsblume.

Von der Tinktur werden
40 Tropfen fünf- bis sechsmal täglich verabreicht.

Durch die Behandlung mit den Tabletten als auch der Tinktur wurde ein deutlich verbessertes Hautbild erhalten. Die Haut zeigte sich weniger entzündet, weicher, glatter, weniger Rötungen sind sichtbar. Insgesamt ist das Hautbild ruhiger und stabiler und nähert sich immer mehr dem Hautbild einer gesunden Person an.

Die Behandlung mit den Tabletten und Tinktur führte für gewöhnlich nach einer 7- bis 14-tägigen Behandlung bereits zu einer deutlichen Verbesserung des Hautbilds. Dies zeigt sich insbesondere bei Neurodermitis, die im Gesicht auftritt. Durch die Behandlung werden ein Abblassen der Rötungen sowie ein Zurückgehen der Entzündungen beobachtet. Die Gesichtshaut scheint sich zu regenerieren und ist im Aussehen nach einigen Tagen kaum noch von der einer gesunden Person zu unterscheiden.

Es wird somit erfindungsgemäß erstmals eine pharmazeutische Zusammensetzung, umfassend einen pflanzlichen und/oder tierischen Wirkstoff zur Behandlung von Neurodermitis durch orale Verabreichung bereitgestellt. Zusätzlich ist auch die topische Anwendung, basierend auf einem tierischen Wirkstoff optional zusammen mit einem pflanzlichen Wirkstoff möglich. Die Wirksamkeit ist überraschend hoch, wobei die Nachteile von klassischen Arzneimitteln nicht auftreten. Vielmehr liegt eine hohe Verträglichkeit bei der Verwendung vor, wobei gleichzeitig die Eigenschaften der Haut sogar positiv beeinflusst werden.

Die Erfindung umfasst Aspekte, die in den nachfolgenden Sätzen offenbart sind, und die Teil der Beschreibung darstellen, aber keine Ansprüche sind.

### Sätze

1. Pharmazeutische Zusammensetzung, umfassend Passionsblumenkraut in Form eines Trockenextrakts als einen Wirkstoff und/oder getrocknetes Miesmuschelfuß-Konzentrat als weiteren Wirkstoff, zur Verwendung in der Behandlung von Neurodermitis durch orale oder topische Verabreichung, mit der Maßgabe, dass Passionsblumenkraut in Form eines Trockenextrakts nur zusammen mit getrocknetem Miesmuschelfuß-Konzentrat topisch verabreicht wird.
2. Pharmazeutische Zusammensetzung nach Satz 1,
wobei die orale Dosierungsform ausgewählt wird aus Tabletten, insbesondere Filmtabletten, Pillen, Dragees, Pastillen, Pellets, Kapseln, insbesondere Hart- oder Weichkapseln, Pulver, Granulaten, Lösungen, insbesondere Tinkturen in Form von Tropfen, Suspensionen, Dispersionen oder Emulsionen, bevorzugt Filmtabletten, Kapseln, Tinkturen in Form von Tropfen.
3. Pharmazeutische Zusammensetzung nach einem der vorangehenden Sätze 1 oder 2,
wobei die topische Dosierungsform ausgewählt wird aus einer Lösung, insbesondere als Tinktur in Form von Tropfen, Öl, Suspension, insbesondere einer Schüttelmixtur, Dispersion, Emulsion, Lotion oder Milch, Creme oder Paste, bevorzugt einer Tinktur in Form von Tropfen oder Creme.
4. Pharmazeutische Zusammensetzung nach einem der vorangehenden Sätze 1 bis 3,
wobei die Menge, je Dosierungsform, zur oralen Verabreichung des Trockenextrakts des Passionsblumenkrauts im Bereich von 45 bis 500 mg oder 90 bis 450 mg oder 100 bis 450 mg oder 150 bis 450 mg oder 200 bis 450 mg oder 250 bis 450 mg liegt.
5. Pharmazeutische Zusammensetzung nach einem der vorangehenden Sätze 1 bis 4,
wobei die Gesamtdosis zur oralen Verabreichung des Trockenextrakts des Passionsblumenkrauts im Bereich von 45 bis 2500 mg oder 90 bis 2250 mg oder 100 bis 2000 mg oder 225 bis 1800 mg oder 400 bis 1500 mg für einen Patienten pro Tag liegt.
6. Pharmazeutische Zusammensetzung nach einem der vorangehenden Sätze 1 bis 5,
wobei die Menge, je Dosierungsform, zur oralen Verabreichung des getrockneten Miesmuschelfuß-Konzentrats im Bereich von 1 mg bis 50 mg oder 1 bis 30 mg oder 1 bis 15 mg oder 2 bis 12 mg oder 3 bis 10 mg liegt.
7. Pharmazeutische Zusammensetzung nach einem der vorangehenden Sätze 1 bis 6,
wobei die Gesamtdosis für das getrocknete Miesmuschelfuß-Konzentrat im Bereich von etwa 5 bis 250 mg oder 10 bis 200 mg oder 15 bis 150 mg oder 15 bis 100 mg für einen Patienten pro Tag liegt.
8. Pharmazeutische Zusammensetzung nach einem der vorangehenden Sätze 1 bis 7,
wobei der Trockenextrakt des Passionsblumenkrauts als ein Wirkstoff und das getrocknete Miesmuschelfuß-Konzentrat als weiterer Wirkstoff als Festdosiskombination verabreicht werden, wobei die beiden Wirkstoffe, gleichzeitig in derselben pharmazeutischen Formulierung vorliegen oder als freie Dosiskombination, wobei die beiden Wirkstoffe, in getrennten pharmazeutischen Formulierungen vorliegen, die gleich oder verschieden sein können.
9. Pharmazeutische Zusammensetzung nach einem der vorangehenden Sätze 1 bis 8,
wobei der Trockenextrakt des Passionsblumenkrauts von Passionsblumen aus biologischem Anbau ist und/oder
dass das getrocknete Miesmuschelfuß-Konzentrat von Miesmuscheln aus einem Naturschutzgebiet ist.
10. Pharmazeutische Zusammensetzung nach einem der vorangehenden Sätze 1 bis 9,
wobei eine orale Dosierungsform
- Passionsblumenkraut in Form eines Trockenextrakts und optional einen oder mehrere pharmazeutisch akzeptable Hilfsstoffe aufweist; oder
- Passionsblumenkraut in Form eines Trockenextrakts und getrocknetes Miesmuschelfuß-Konzentrat und optional einen oder mehrere pharmazeutisch akzeptable Hilfsstoffe aufweist; oder
- getrocknetes Miesmuschelfuß-Konzentrat und optional einen oder mehrere pharmazeutisch akzeptable Hilfsstoffe aufweist.
11. Pharmazeutische Zusammensetzung nach einem der vorangehenden Sätze 1 bis 9,
wobei eine topische Dosierungsform getrocknetes Miesmuschelfuß-Konzentrat aufweist und gegebenenfalls Passionsblumenkraut in Form eines Trockenextrakts sowie einen oder mehrere pharmazeutisch akzeptable Hilfsstoffe.
12. Pharmazeutische Zusammensetzung nach einem der vorangehenden Sätze 10 oder 11,
wobei nur vegane Hilfsstoffe, insbesondere nur pflanzliche Hilfsstoffe eingesetzt werden.
13. Pharmazeutische Zusammensetzung nach einem der vorangehenden Sätze 1 bis 12,
wobei eine Dosierungsform ausgewählt wird aus:
einer Kapsel, umfassend

| | |
|---|---|
| - Trockenextrakt aus Passionsblumenkraut: | 5,0 - 99,9 Gew.-% |

und

| | |
|---|---|
| - getrocknetes Miesmuschelfuß-Konzentrat: | 0,1 - 10,0 Gew.-%, |

oder

| | |
|---|---|
| - Trockenextrakt aus Passionsblumenkraut: | 5,0 - 100 Gew.-% |

oder

| | |
|---|---|
| - getrocknetes Miesmuschelfuß-Konzentrat: | 0,1 - 100 Gew.-% |

und optional die folgenden Hilfsstoffe:

| | |
|---|---|
| - Lactose-Monohydrat | 0 - 45,0 Gew.-% |
| - Cellulosepulver | 0 - 45,0 Gew.-% |
| - hochdisperses Siliciumdioxid | 0 - 5,0 Gew.-% |
| - pflanzliches Magnesiumstearat | 0 - 5,0 Gew.-% |
| - vegane Gelatine aus Rotalgen | 0 - 5,0 Gew.-% |
| - Natriumdodecylsulfat | 0 - 5,0 Gew.-% |
| - Chlorophyllin-Kupfer-Komplex (E 141) | 0 - 2,0 Gew.-% |
| - Titandioxid (E171) | 0 - 5,0 Gew.-%, |

wobei die Mengen der jeweiligen Komponenten so gewählt sind, dass das Gesamtgewicht der Komponenten 100 Gew.-% nicht übersteigt; oder
einer Tablette, umfassend

| | |
|---|---|
| - Trockenextrakt aus Passionsblumenkraut: | 5,0 - 93,4 Gew.-% |

und

| | |
|---|---|
| - getrocknetes Miesmuschelfuß-Konzentrat: | 0,1 - 10 Gew.-%, |

oder

| | |
|---|---|
| - Trockenextrakt aus Passionsblumenkraut: | 5,0 - 93,5 Gew.-% |

oder

| | |
|---|---|
| - getrocknetes Miesmuschelfuß-Konzentrat: | 0,1 - 93,5 Gew.-% |

und die folgenden Hilfsstoffe:

| | |
|---|---|
| - Lactose-Monohydrat | 2,5 - 45,0 Gew.-% |
| - Cellulosepulver | 3,5 - 45,0 Gew.-% |
| - hochdisperses Siliciumdioxid | 0 - 5,0 Gew.-% |
| - pflanzliches Magnesiumstearat | 0,5 - 5,0 Gew.-% |
| - vegane Gelatine aus Rotalgen | 0 - 5,0 Gew.-% |
| - Natriumdodecylsulfat | 0 - 5,0 Gew.-% |
| - Chlorophyllin-Kupfer-Komplex (E 141) | 0 - 2,0 Gew.-% |
| - Titandioxid (E171) | 0 - 5,0 Gew.-%, |

wobei die Mengen der jeweiligen Komponenten so gewählt sind, dass das Gesamtgewicht der Komponenten 100 Gew.-% nicht übersteigt;
sowie optional eine zusätzliche Beschichtung der Tablette; oder
einer Tinktur zur oralen Verabreichung, umfassend
10 - 100 g Trockenextrakt von Passionsblumenkraut und/oder
0,20 bis 6,0 g Miesmuschelfuß-Konzentrat,
mit einem Lösungsmittel auf 200 g Lösung aufgefüllt, wobei das Lösungsmittel ausgewählt ist aus Wasser oder einer Alkohol/Wasser-Mischung mit einem Verhältnis von 1 : 1 bis 10; oder
einer Tinktur zur topischen Verabreichung, umfassend
0,20 bis 6,0 g Miesmuschelfuß-Konzentrat und
optional 10 - 100 g Trockenextrakt von Passionsblumenkraut,
mit einem Lösungsmittel auf 200 g Lösung aufgefüllt, wobei das Lösungsmittel ausgewählt ist aus Wasser oder einer Alkohol/Wasser-Mischung mit einem Verhältnis von 1 : 1 bis 10; oder
einer Creme, umfassend

| | |
|---|---|
| getrocknetes Miesmuschelfuß-Konzentrat | 0,5 - 10,00 Gew.-% |

und

| | |
|---|---|
| optional Trockenextrakt von Passionsblumenkraut sowie | 0 - 50,00 Gew.-% |
| Jojobaöl | 5,00 - 25,00 Gew.-% |
| Mandelöl | 1,00 - 15,00 Gew.-% |
| Olivenöl | 1,00 - 8,00 Gew.-% |
| Temulgator (pflanzlicher Emulgator aus Palmöl) | 1,00 - 10,00 Gew.-% |
| Sheabutter (bevorzugt raffiniert) | 1,00 - 10,00 Gew.-% |
| D-alpha-Tocopherylsuccinat (Vitamin E) | 0,10 - 2,00 Gew.-% |
| Phenetylalkohol | 0,10 - 1,00 Gew.-% |
| ätherische(s) Öl(e) | 0,10 - 2,00 Gew.-% |
| destilliertes Wasser | auf 100 Gew.-%, |

wobei die Mengen der jeweiligen Komponenten so gewählt sind, dass das Gesamtgewicht der Komponenten 100 Gew.-% nicht übersteigt.

## Patentansprüche

1. Pharmazeutische Zusammensetzung, umfassend
Passionsblumenkraut in Form eines Trockenextrakts als einen Wirkstoff und/oder getrocknetes Miesmuschelfuß-Konzentrat als weiteren Wirkstoff,
zur Verwendung in der Behandlung von Neurodermitis durch orale oder topische Verabreichung,
wobei das Passionsblumenkraut in Form eines Trockenextrakts erhältlich ist durch
- Ernten, Zerkleinern und Trocknen des Passionsblumenkrauts in Form der Pflanzenteile der Passionsblume, ohne Wurzel;
- Durchführen einer Fest-Flüssig-Extraktion der getrockneten Pflanzenteile unter Verwendung von einem Extraktionsmittel, ausgewählt aus Wasser, Alkohol oder Alkohol-Wasser-Gemischen bei Raumtemperatur oder einer Temperatur im Bereich zwischen Raumtemperatur und 78°C, mit oder ohne Rühren und/oder Schütteln;
- Abtrennen der Pflanzenteile vom Extraktionsmittel durch Dekantieren oder Filtrieren, und
- Abdampfen des Extraktionsmittels unter Erhalt eines Trockenextrakts des Passionsblumenkrauts; und
wobei das getrocknete Miesmuschelfuß-Konzentrat zerkleinerten und getrockneten Miesmuschelfuß von Miesmuscheln darstellt,
mit der Maßgabe, dass Passionsblumenkraut in Form eines Trockenextrakts nur zusammen mit getrocknetem Miesmuschelfuß-Konzentrat topisch verabreicht wird und dass Candida albicans und N,N-Dimethylglycin in der pharmazeutischen Zusammensetzung nicht enthalten sind.

2. Pharmazeutische Zusammensetzung nach Anspruch 1,
**dadurch gekennzeichnet,**
**dass** die orale Dosierungsform ausgewählt wird aus Tabletten, insbesondere Filmtabletten, Pillen, Dragees, Pastillen, Pellets, Kapseln, insbesondere Hart- oder Weichkapseln, Pulver, Granulaten, Lösungen, insbesondere Tinkturen in Form von Tropfen, Suspensionen, Dispersionen oder Emulsionen, bevorzugt Filmtabletten, Kapseln, Tinkturen in Form von Tropfen.

3. Pharmazeutische Zusammensetzung nach einem der vorangehenden Ansprüche 1 oder 2,
**dadurch gekennzeichnet,**
**dass** die topische Dosierungsform ausgewählt wird aus einer Lösung, insbesondere als Tinktur in Form von Tropfen, Öl, Suspension, insbesondere einer Schüttelmixtur, Dispersion, Emulsion, Lotion oder Milch, Creme oder Paste, bevorzugt einer Tinktur in Form von Tropfen oder Creme.

4. Pharmazeutische Zusammensetzung nach einem der vorangehenden Ansprüche 1 bis 3,
**dadurch gekennzeichnet,**
**dass** die Menge, je Dosierungsform, zur oralen Verabreichung des Trockenextrakts des Passionsblumenkrauts im Bereich von 45 bis 500 mg oder 90 bis 450 mg oder 100 bis 450 mg oder 150 bis 450 mg oder 200 bis 450 mg oder 250 bis 450 mg liegt.

5. Pharmazeutische Zusammensetzung nach einem der vorangehenden Ansprüche 1 bis 4,
**dadurch gekennzeichnet,**
**dass** die Gesamtdosis zur oralen Verabreichung des Trockenextrakts des Passionsblumenkrauts im Bereich von 45 bis 2500 mg oder 90 bis 2250 mg oder 100 bis 2000 mg oder 225 bis 1800 mg oder 400 bis 1500 mg für einen Patienten pro Tag liegt.

6. Pharmazeutische Zusammensetzung nach einem der vorangehenden Ansprüche 1 bis 5,
**dadurch gekennzeichnet,**
**dass** die Menge, je Dosierungsform, zur oralen Verabreichung des getrockneten Miesmuschelfuß-Konzentrats im Bereich von 1 mg bis 50 mg oder 1 bis 30 mg oder 1 bis 15 mg oder 2 bis 12 mg oder 3 bis 10 mg liegt.

7. Pharmazeutische Zusammensetzung nach einem der vorangehenden Ansprüche 1 bis 6,
**dadurch gekennzeichnet,**
**dass** die Gesamtdosis für das getrocknete Miesmuschelfuß-Konzentrat im Bereich von etwa 5 bis 250 mg oder 10 bis 200 mg oder 15 bis 150 mg oder 15 bis 100 mg für einen Patienten pro Tag liegt.

8. Pharmazeutische Zusammensetzung nach einem der vorangehenden Ansprüche 1 bis 7,
**dadurch gekennzeichnet,**
**dass** der Trockenextrakt des Passionsblumenkrauts als ein Wirkstoff und das getrocknete Miesmuschelfuß-Konzentrat als weiterer Wirkstoff als Festdosiskombination verabreicht werden, wobei die beiden Wirkstoffe, gleichzeitig in derselben pharmazeutischen Formulierung vorliegen oder als freie Dosiskombination, wobei die beiden Wirkstoffe, in getrennten pharmazeutischen Formulierungen vorliegen, die gleich oder verschieden sein können.

9. Pharmazeutische Zusammensetzung nach einem der vorangehenden Ansprüche 1 bis 8,
**dadurch gekennzeichnet,**
**dass** der Trockenextrakt des Passionsblumenkrauts von Passionsblumen aus biologischem Anbau ist und/oder
**dass** das getrocknete Miesmuschelfuß-Konzentrat von Miesmuscheln aus einem Naturschutzgebiet ist.

10. Pharmazeutische Zusammensetzung nach einem der vorangehenden Ansprüche 1 bis 9,
**dadurch gekennzeichnet,**
**dass** eine orale Dosierungsform
- Passionsblumenkraut in Form eines Trockenextrakts und optional einen oder mehrere pharmazeutisch akzeptable Hilfsstoffe aufweist; oder
- Passionsblumenkraut in Form eines Trockenextrakts und getrocknetes Miesmuschelfuß-Konzentrat und optional einen oder mehrere pharmazeutisch akzeptable Hilfsstoffe aufweist; oder
- getrocknetes Miesmuschelfuß-Konzentrat und optional einen oder mehrere pharmazeutisch akzeptable Hilfsstoffe aufweist.

11. Pharmazeutische Zusammensetzung nach einem der vorangehenden Ansprüche 1 bis 9,
**dadurch gekennzeichnet,**
**dass** eine topische Dosierungsform getrocknetes Miesmuschelfuß-Konzentrat aufweist und gegebenenfalls Passionsblumenkraut in Form eines Trockenextrakts sowie einen oder mehrere pharmazeutisch akzeptable Hilfsstoffe.

12. Pharmazeutische Zusammensetzung nach einem der vorangehenden Ansprüche 10 oder 11,
**dadurch gekennzeichnet,**
**dass** nur vegane Hilfsstoffe, insbesondere nur pflanzliche Hilfsstoffe eingesetzt werden.

13. Pharmazeutische Zusammensetzung nach einem der vorangehenden Ansprüche 1 bis 12,
**dadurch gekennzeichnet,**
**dass** eine Dosierungsform ausgewählt wird aus:
einer Kapsel, umfassend
| | |
|---|---|
| - Trockenextrakt aus Passionsblumenkraut: | 5,0 - 99,9 Gew.-% |
und
| | |
|---|---|
| - getrocknetes Miesmuschelfuß-Konzentrat: | 0,1 - 10,0 Gew.-%, |
oder
| | |
|---|---|
| - Trockenextrakt aus Passionsblumenkraut: | 5,0 - 100 Gew.-% |
oder
| | |
|---|---|
| - getrocknetes Miesmuschelfuß-Konzentrat: | 0,1 - 100 Gew.-% |
und optional die folgenden Hilfsstoffe:
| | |
|---|---|
| - Lactose-Monohydrat | 0 - 45,0 Gew.-% |
| - Cellulosepulver | 0 - 45,0 Gew.-% |
| - hochdisperses Siliciumdioxid | 0 - 5,0 Gew.-% |
| - pflanzliches Magnesiumstearat | 0 - 5,0 Gew.-% |
| - vegane Gelatine aus Rotalgen | 0 - 5,0 Gew.-% |
| - Natriumdodecylsulfat | 0 - 5,0 Gew.-% |
| - Chlorophyllin-Kupfer-Komplex (E 141) | 0 - 2,0 Gew.-% |
| - Titandioxid (E171) | 0 - 5,0 Gew.-%, |
wobei die Mengen der jeweiligen Komponenten so gewählt sind, dass das Gesamtgewicht der Komponenten 100 Gew.-% nicht übersteigt; oder
einer Tablette, umfassend
| | |
|---|---|
| - Trockenextrakt aus Passionsblumenkraut: | 5,0 - 93,4 Gew.-% |
und
| | |
|---|---|
| - getrocknetes Miesmuschelfuß-Konzentrat: | 0,1 - 10 Gew.-%, |
oder
| | |
|---|---|
| - Trockenextrakt aus Passionsblumenkraut: | 5,0 - 93,5 Gew.-% |
oder
| | |
|---|---|
| - getrocknetes Miesmuschelfuß-Konzentrat: | 0,1 - 93,5 Gew.-% |
und die folgenden Hilfsstoffe:
| | |
|---|---|
| - Lactose-Monohydrat | 2,5 - 45,0 Gew.-% |
| - Cellulosepulver | 3,5 - 45,0 Gew.-% |
| - hochdisperses Siliciumdioxid | 0 - 5,0 Gew.-% |
| - pflanzliches Magnesiumstearat | 0,5 - 5,0 Gew.-% |
| - vegane Gelatine aus Rotalgen | 0 - 5,0 Gew.-% |
| - Natriumdodecylsulfat | 0 - 5,0 Gew.-% |
| - Chlorophyllin-Kupfer-Komplex (E 141) | 0 - 2,0 Gew.-% |
| - Titandioxid (E171) | 0 - 5,0 Gew.-%, |
wobei die Mengen der jeweiligen Komponenten so gewählt sind, dass das Gesamtgewicht der Komponenten 100 Gew.-% nicht übersteigt;
sowie optional eine zusätzliche Beschichtung der Tablette; oder
einer Tinktur zur oralen Verabreichung, umfassend
10 - 100 g Trockenextrakt von Passionsblumenkraut und/oder
0,20 bis 6,0 g Miesmuschelfuß-Konzentrat,
mit einem Lösungsmittel auf 200 g Lösung aufgefüllt, wobei das Lösungsmittel ausgewählt ist aus Wasser oder einer Alkohol/Wasser-Mischung mit einem Verhältnis von 1 : 1 bis 10; oder
einer Tinktur zur topischen Verabreichung, umfassend
0,20 bis 6,0 g Miesmuschelfuß-Konzentrat und
optional 10 - 100 g Trockenextrakt von Passionsblumenkraut,
mit einem Lösungsmittel auf 200 g Lösung aufgefüllt, wobei das Lösungsmittel ausgewählt ist aus Wasser oder einer Alkohol/Wasser-Mischung mit einem Verhältnis von 1 : 1 bis 10; oder
einer Creme, umfassend
| | |
|---|---|
| getrocknetes Miesmuschelfuß-Konzentrat | 0,5 - 10,00 Gew.-% |
und
| | |
|---|---|
| optional Trockenextrakt von Passionsblumenkraut sowie | 0 - 50,00 Gew.-% |
| Jojobaöl | 5,00 - 25,00 Gew.-% |
| Mandelöl | 1,00 - 15,00 Gew.-% |
| Olivenöl | 1,00 - 8,00 Gew.-% |
| Temulgator (pflanzlicher Emulgator aus Palmöl) | 1,00 - 10,00 Gew.-% |
| Sheabutter (bevorzugt raffiniert) | 1,00 - 10,00 Gew.-% |
| D-alpha-Tocopherylsuccinat (Vitamin E) | 0,10 - 2,00 Gew.-% |
| Phenetylalkohol | 0,10 - 1,00 Gew.-% |
| ätherische(s) Öl(e) | 0,10 - 2,00 Gew.-% |
| destilliertes Wasser | auf 100 Gew.-%, |
wobei die Mengen der jeweiligen Komponenten so gewählt sind, dass das Gesamtgewicht der Komponenten 100 Gew.-% nicht übersteigt.
